# EUROPEAN PATENT SPECIFICATION

(11) **EP 2 377 943 B1**
(45) Date of publication and mention of the grant of the patent: **17.02.2016**
(21) Application number: 11162906.9
(22) Date of filing: 18.04.2011
(51) Int. Cl.: C12P 7/10, C12P 19/02, C12P 19/14, C12P 21/02, C12N 1/38, C12N 9/42

(54) **Extraction of solubles from plant biomass for use as a growth stimulant and methods related thereto**
Extraktion löslicher Stoffe aus pflanzlicher Biomasse zur Verwendung als Wachstumsförderer, sowie zugehörige Methoden
Extraction de composés solubles à partir de biomasse végétale et son utilisation comme facteur de croissance, ainsi que des méthodes associées

(30) Priority: 19.04.2010 US 763102
(43) Date of publication of application: 19.10.2011
(73) Proprietor: Board Of Trustees Of Michigan State University, East Lansing, Michigan 48824-1046 (US)
(72) Inventor: Lau, Ming Woei, Maryville, TN 37804 (US); Dale, Bruce E., Mason, MI 48854 (US); Balan, Venkatesh, East Lansing, MI 48823 (US); Chundawat, Shishir, Piscataway, NJ 08854 (US)
(74) Representative: Danner, Stefan

(56) References cited:
- EP-A1- 1 690 944
- WO-A2-2007/005918
- WO-A2-2008/020901
- US-A1- 2010 267 999
- CHINEDU-NWODO S ET AL: "Xylanase production of Aspergillus niger and Penicillium chrysogenum from ammonia pretreated cellulosic waste", RESEARCH JOURNAL OF MICROBIOLOGY, [Online] vol. 3, no. 4, April 2008 (2008-04), pages 246-253, XP002656733, ISSN: 1816-4935 Retrieved from the Internet: URL:http://scialert.net/qredirect.php?doi= jm.2008.246.253&linkid=pdf> [retrieved on 2011-08-10]
- Jain, A and Walker, T H: "Effect of ammonia pretreatment on switchgrass for production of cellulase using Trichoderma reesei Rut C-30", 31st Symposium onbiotechnology for fuels and chemicals, Poster session 2, 4 May 2009 (2009-05-04), pages 1-1, XP002656737, Retrieved from the Internet: URL:http://sim.confex.com/sim/31st/techpro gram/P8269.HTM [retrieved on 2011-08-11]
- PEILIN CEN LIMING XIA: "Production of cellulase by solid-state fermentation", ADVANCES IN BIOCHEMICAL ENGINEERING, BIOTECHNOLOGY, SPRINGER, BERLIN, DE, 1 January 1990 (1990-01-01), pages 70-92, XP009107560, ISSN: 0724-6145
- LYND LEE R ET AL: "Microbial cellulose utilization: fundamentals and biotechnology", MICROBIOLOGY AND MOLECULAR BIOLOGY REVIEWS, AMERICAN SOCIETY FOR MICROBIOLOGY, US, vol. 66, no. 3, 1 September 2002 (2002-09-01), pages 506-577, XP002551605, ISSN: 1092-2172, DOI: 10.1128/MMBR.66.3.506-577.2002
- WARZYWODA M ET AL: "Production and characterization of cellulolytic enzymes from Trichoderma reesei grown on various carbon sources", BIORESOURCE TECHNOLOGY, ELSEVIER BV, GB, vol. 39, no. 2, 1 January 1992 (1992-01-01), pages 125-130, XP008054567, ISSN: 0960-8524, DOI: 10.1016/0960-8524(92)90130-P
- CHAHAL P S ET AL: "Production of cellulase in solid-state fermentation with Trichoderma reesei MCG 80 on wheat straw", APPLIED BIOCHEMISTRY AND BIOTECHNOLOGY, THE HUMANA PRESS, INC, US, vol. 57/58, 1 January 1996 (1996-01-01), pages 433-442, XP008054568, ISSN: 0273-2289, DOI: 10.1007/BF02941724
- SINGHANIA R R ET AL: "Advancement and comparative profiles in the production technologies using solid-state and submerged fermentation for microbial cellulases", ENZYME AND MICROBIAL TECHNOLOGY, STONEHAM, MA, US, [Online] vol. 46, no. 7, 31 March 2010 (2010-03-31) , pages 541-549, XP027038189, ISSN: 0141-0229

## Description

### Background

Cellulosic biomass can be used for the production of various bio-products. However, many conventional methods are very expensive, requiring high capital expenditures, such as for high pressure reactors and large amounts of additives.

For example, Chinedu and coworkers (Chinedu, N.S. et al. (2008) Res. J. Microbiol. 3, 246-253) describe the effect of ammonia pretreatment of cellulosic wastes on xylanase production of *Aspergillus niger* and *Penicillium chrysogenum.*

International patent publication WO 2007/005918 A2 relates to a process of producing cellulase in a host cell, wherein ammonia pretreated lingo-cellulosic material is added to induce cellulase production. The cellulase can be secreted into the fermentation medium or remain intracellularly.

Jain and Walker (Jain, A. and Walker, T.H. (2009) 31st Symposium on Biotechnology for Fuels and Chemicals*.* Abstract 5-34, Poster Session 2) describe the effect of ammonia pretreatment on switchgrass for the production cellulose using *Trichoderma reesei.*

The review article of Cen and Xia (Cen, P. and Xia, L. (1999) Adv. Biochem. Engineer. 65, 69-92) summarizes methods for the production of cellulase by solid-state fermentation.

European patent application 1 690 944 A1 discloses the production of cellulases from lignocellulosic biomass, wherein the pre-treatment step is performed by steam-explosion under acid conditions.

Warzywoda, M. et al. (Warzywoda, M. et al. (1992) Bioresource Technol. 39, 125-130) report on the production and characterization of cellulolytic enzymes from *Trichoderma reesei* grown on various carbon sources.

The inventors recognize the need for improved methods of producing bio-products from cellulosic biomass.

### Summary

One aspect of the invention is a method as specified in claim 1, comprising: treating plant biomass with an ammonia pretreatment to provide an ammonia-pretreated plant biomass, wherein the plant biomass is from one or more plants, and wherein the biomass contains solubles, or solubles and solids; extracting at least a portion of the solubles from the ammonia-pretreated plant biomass with a solvent to produce a soluble extract and an extracted ammonia-pretreated plant biomass, wherein the solvent is water; and using the soluble extract as a stimulant to stimulate growth and promote cellulase and/or hemicellulase enzyme synthesis in an enzyme-producing microorganism.

The methods described herein can be performed in a bio-product production facility that utilizes the extracted ammonia-pretreated plant biomass to produce a bio-product in a bio-product production unit. In some embodiments, the enzyme-producing microorganism is contained within an enzyme production unit that is in communication with the bio-product unit and that optionally comprises the soluble extract, the extracted pretreated biomass and/or a nutrient-containing byproduct of the bio-product production unit.

The enzyme produced by the enzyme-producing microorganism is cellulase and/or hemicellulase. For example, the cellulase and/or hemicellulase can be selected from exoglucanases, endoglucanases, β-xylosidases, endoxylanases, α-arabinofuranosidases, cellulose induced protein 2, α-arabinofuranosidases, acetyl xylan esterases, α-glucuronidases, endoxylanases, polygalaturonases, α-galactosidases, acetyl esterases, and combinations thereof.

The enzyme-producing microorganism is selected from yeast, bacteria, fungi, and combinations thereof. In some embodiments, the enzyme-producing microorganism can be selected from *Trichoderma reesei (T. reesei)* and *Aspergillus awamori (A. awamori).* In some embodiments, the enzyme-producing microorganism can be selected from *T. reesei, A. awamori, Clostridium thermocellum (C. thermocellum),* and *Thermoanaerobacterium saccharolyticum.*

The soluble extract obtained from the ammonia-treated alkaline plant biomass can include proteins, vitamins, simple sugars, oligosaccharides, lipids and trace elements.

The solvent used for extracting at least a portion of the solubles from the ammonia-treated alkaline plant biomass is water.The soluble extract is used as a stimulant to stimulate growth in an enzyme-producing microorganism and to induce or stimulate cellulase and/or hemicellulase enzyme production in an enzyme-producing microorganism. Such growth stimulation and enzyme induction can be performed separately or simultaneously, for example, in an enzyme production unit.

The plant biomass is derived from one or more plants. In some embodiments the plant is a monocot. Examples of a monocot from which the plant biomass can be obtained include grass, corn stover, sorghum, sugarcane bagasse, wheat, rice, maize, or a combination thereof. In some embodiments, the grass is switchgrass, miscanthus, reed canary grass, or a combination thereof. In one embodiment, the plant biomass is corn stover.

The ammonia pretreatment can be performed in a variety of ways using a variety of ammonia-containing material. In some embodiments, the ammonia pretreatment comprises ammonia fiber expansion.

The method described herein can further include hydrolyzing the ammonia-treated plant biomass with an enzyme to generate an enzymatic hydrolysate slurry, wherein the enzyme is an enzyme secreted by the enzyme-producing microorganism.

The enzymatic hydrolysate slurry can be mechanically processed to produce a liquid enzymatic hydrolysate that includes a fermentable sugar mixture.

The methods described herein can further include fermenting the liquid enzymatic hydrolysate to generate a bio-product.

Bio-based products are produced by the methods described herein. For example, the bio-based product generated by the methods described herein can be a biofuel, hydrocarbon or a biochemical product.

In one embodiment, the method described herein is performed in a bioproduct production facility that includes: a bio-product production unit configured to produce a bio-product from the extracted ammonia pretreated biomass; and an enzyme production unit in communication with the bio-product production unit and containing the enzyme-producing microorganism, and optionally comprising the extracted ammonia-treated biomass, a nutrient-containing byproduct and/or the soluble extract of the bio-product production unit.

The bioproduct production facility produces a bio-product such as a biofuel, hydrocarbon, biochemical or a combination thereof. In some embodiments, the biofuel is an alcohol (e.g., ethanol). The ammonia pretreated biomass used in the biofuel production facility can be ammonia pretreated corn stover.

In another embodiment, the method of producing a bio-product comprises: producing an enzyme in an enzyme production unit comprising an enzyme-producing microorganism, an ethanol production residue, and a stimulant extracted from an ammonia pretreated biomass; and providing the enzyme to a bio-product production unit wherein the bio-product is produced. For example, the ammonia pretreated biomass from which the stimulant is extracted can be ammonia treated corn stover. In some embodiments, the ethanol production residue is corn syrup liquor. The ethanol production residue can, for example, be a nutrient-containing bioproduct generated in the bio-product production unit. Moreover, the enzyme production unit can include a dilute pretreated biomass. The bio-product produced in the method can be a biochemical or biofuel (e.g., an alcohol).

The stimulant extracted from an ammonia pretreated biomass can include solubles capable of stimulating microbial growth. The stimulant can also contain solubles capable of stimulating or inducing enzyme production in the enzyme-producing microorganism. The enzyme produced is a cellulase and/or hemicellulase. For example, the saccharolytic enzyme can be selected from exoglucanases, endoglucanases, β-xylosidases, endoxylanases, α-arabinofuranosidases, cellulose induced protein 2, α-arabinofuranosidases, acetyl xylan esterases, α-glucuronidases, endoxylanases, polygalaturonases, α-galactosidases, acetyl esterases, and combinations thereof.

In some embodiments, the enzyme-producing microorganism in the enzyme production unit can be selected from *T. reesei* and *A. awamori.* In some embodiments, the enzyme-producing microorganism in the enzyme production unit can be selected from *T. reesei, A. awamori, C. thermocellum* and *Thermoanaerobacterium saccharolyticum.*

### Brief Description of the Drawings

FIG. 1A is a process flow diagram of an integrated bio-product production facility employed according to various embodiments.
FIG. 1B is a process flow diagram of a portion of the integrated bio-product production facility of FIG. 1A employed according to various embodiments.
FIG. 2A is a graph showing the effect of extraction temperature on protein and biomass solubilization yields for Ammonia Fiber Expansion (AFEX) treated switchgrass (AFEX-SG) according to various embodiments.
FIG. 2B is a graph showing the effect of ammonia concentration on protein and biomass solubilization yields for AFEX-SG according to various embodiments.
FIG. 3A is a graph showing the effect of extraction pH on protein and biomass solubilization yields for AFEX-SG according to various embodiments.
FIG. 3B is a graph showing the effect of reducing agents on protein solubilization yields for untreated switchgrass and AFEX-SG according to various embodiments.
FIG. 4 is a graph showing amino acid profiles for untreated switchgrass protein, AFEX-SG protein, and native switchgrass protein according to various embodiments.
FIG. 5 is a process flow diagram for AFEX treatment with extraction prior to hydrolysis according to various embodiments.
FIG. 6 is a process flow diagram for AFEX treatment with extraction after hydrolysis according to various embodiments.
FIG. 7 shows exemplary components input to and resulting from a 6% cellulose loading solids loading enzymatic hydrolysis according to various embodiments.
FIG. 8 shows an exemplary mass balance for the process shown in FIG. 8, including a mass balance for components input to and output from an AFEX treatment process according to various embodiments.
FIG. 9A is a graph showing glucose consumption in fermentation of 9% Solids Loading Equivalent (SLE) water extract from AFEX-treated corn stover (AFEX-CS) and AFEX-treated rice straw (AFEX-RS) according to various embodiments.
FIG. 9B is a graph showing cell density in fermentation of 9% SLE water extract from AFEX-CS and AFEX-RS according to various embodiments.
FIG. 9C is a schematic representation of two-stage ethanol fermentation and native S. *cerevisiae* co-product generation according to various embodiments.
FIG. 9D is a schematic representation of cell recycling of recombinant S. *cerevisiae* for high cell density xylose fermentation according to various embodiments.
FIG. 10A is a graph showing sugar and ethanol profiles for two-stage fermentation according to various embodiments.
FIG. 10B is a graph showing xylose consumption by recombinant S. *cerevisiae* 424A (LNH-ST) over three generations of recycling according to various embodiments.
FIG. 11A is a schematic illustration of enzyme production using *T. reseei* RUT-C30 fermentation according to various embodiments.
FIG. 11B is a graph comparing the relative activity of enzymes induced using AFEX-CS and lactose according to various embodiments.
FIG. 11C is a graph showing net sugar yield of enzymatic hydrolysis on 1% cellulose loading AFEX-CS using 1:6 diluted RUT-C30 broth induced by AFEX-CS mixture according to various embodiments.
FIG. 11D is a graph showing the top 11 secreted *T. reesei* (RUT-C30) cellulases and hemicellulases differentially expressed during induction using AFEX-CS water extract ("WE"), AFEX-CS solid biomass + its water extract ("AFCS + WE") and lactose according to various embodiments.
FIG. 12A is a graph showing concentrations of monomeric sugars and ethanol over time for AFEX-CS hydrolysate fermented using *T. saccharolyticum* ALK2 according to various embodiments.
FIG. 12B is a graph showing sugar concentrations for various monomeric and oligomeric sugars over time for AFEX-CS hydrolysate fermented using *T. saccharolyticum* ALK2 according to various embodiments.
FIG. 13 is a process flow diagram of a biological conversion section of a biorefinery with in-house enzyme production and yeast co-production included according to an embodiment.
FIG. 14A is a bar graph showing cost and revenue breakdown for a model integrated scheme as compared with a model conventional scheme according to an embodiment.
FIG. 14B is a bar graph showing costs and revenues of four types of integrated schemes according to various embodiments.
FIG. 14C is a bar graph showing economic impact of various integrated schemes according to various embodiments.

### Detailed Description of the Embodiments

In the following detailed description of the preferred embodiments, reference is made to the accompanying drawings, which form a part hereof, and in which is shown by way of illustration specific preferred embodiments in which the invention may be practiced. These embodiments are described in sufficient detail to enable those skilled in the art to practice the invention. The following detailed description is, therefore, not to be taken in a limiting sense, and the scope of the present invention is defined only by the appended claims.

The Detailed Description that follows begins with a definition section followed by a brief overview of current technologies for production of commercial products from cellulosic-based biomass, a description of the embodiments, an example section and a brief conclusion.

### Definitions

The term "biomass" as used herein, refers in general to organic matter harvested or collected from a renewable biological resource as a source of energy. The renewable biological resource can include plant materials, animal materials, and/or materials produced biologically. The term "biomass" is not considered to include fossil fuels, which are not renewable.

The term "plant biomass" or "lignocellulosic biomass" or "cellulosic biomass" as used herein, is intended to refer to virtually any plant-derived organic matter (woody or non-woody) available for energy on a sustainable basis. Plant biomass can includeagricultural crop wastes and residues such as corn stover, wheat straw, rice straw, sugar cane bagasse and the like. Plant biomass further includes woody energy crops, wood wastes and residues such as trees, including fruit trees, such as fruit-bearing trees, (e.g., apple trees, orange trees, and the like), softwood forest thinnings, barky wastes, sawdust, paper and pulp industry waste streams, wood fiber, and the like. Additionally grass crops, such as various prairie grasses, including prairie cord grass, switchgrass, miscanthus, big bluestem, little bluestem, side oats grama, and the like, have potential to be produced large-scale as additional plant biomass sources. For urban areas, potential plant biomass feedstock includes yard waste (e.g., grass clippings, leaves, tree clippings, brush, etc.) and vegetable processing waste. Plant biomass is known to be the most prevalent form of carbohydrate available in nature and corn stover is currently the largest source of readily available plant biomass in the United States.

The term "biofuel" as used herein, refers to any renewable solid, liquid or gaseous fuel produced biologically, for example, those derived from biomass. Most biofuels are originally derived from biological processes such as the photosynthesis process and can therefore be considered a solar or chemical energy source. Other biofuels, such as natural polymers (e.g., chitin or certain sources of microbial cellulose), are not synthesized during photosynthesis, but can nonetheless be considered a biofuel because they are biodegradable. There are generally considered to be three types of biofuels derived from biomass synthesized during photosynthesis, namely, agricultural biofuels (defined below), municipal waste biofuels (residential and light commercial garbage or refuse, with most of the recyclable materials such as glass and metal removed) and forestry biofuels (e.g., trees, waste or byproduct streams from wood products, wood fiber, pulp and paper industries). Biofuels produced from biomass not synthesized during photosynthesis include those derived from chitin, which is a chemically modified form of cellulose known as an N-acetyl glucosamine polymer. Chitin is a significant component of the waste produced by the aquaculture industry because it comprises the shells of seafood.

The term "agricultural biofuel", as used herein, refers to a biofuel derived from agricultural crops (e.g., grains, such as corn), crop residues, grain processing facility wastes (e.g., wheat/oat hulls, corn/bean fines, out-of-specification materials, etc.), livestock production facility waste (e.g., manure, carcasses, etc.), livestock processing facility waste (e.g., undesirable parts, cleansing streams, contaminated materials, etc.), food processing facility waste (e.g., separated waste streams such as grease, fat, stems, shells, intermediate process residue, rinse/cleansing streams, etc.), value-added agricultural facility byproducts (e.g., distiller's wet grain (DWG) and syrup from ethanol production facilities, etc.), and the like. Examples of livestock industries include beef, pork, turkey, chicken, egg and dairy facilities. Examples of agricultural crops include any type of non-woody plant (e.g., cotton), grains such as corn, wheat, soybeans, sorghum, barley, oats, rye, and the like, herbs (e.g., peanuts), short rotation herbaceous crops such as switchgrass, alfalfa, and so forth.

The term "pretreatment step" as used herein, refers to any step intended to alter native biomass so it can be more efficiently and economically converted to reactive intermediate chemical compounds such as sugars, organic acids, etc., which can then be further processed into a variety of value added products such as value-added chemicals, such as ethanol. Pretreatment can influence the degree of crystallinity of a polymeric substrate, reduce the interference of lignin with biomass conversion and pre-hydrolyze some of the structural carbohydrates, thus increasing their enzymatic digestibility and accelerating the degradation of biomass to useful products. Pretreatment methods can utilize acids of varying concentrations (including sulfuric acids, hydrochloric acids, organic acids, etc.) and/or other components such as ammonia, ammonium hydroxide, lime, and the like. Pretreatment methods can additionally or alternatively utilize hydrothermal treatments including water, heat, steam or pressurized steam. Pretreatment can occur or be deployed in various types of containers, reactors, pipes, flow through cells and the like. Most pretreatment methods will cause the partial or full solubilization and/or destabilization of lignin and/or hydrolysis of hemicellulose to pentose sugar monomers or oligomers.

The term "moisture content" as used herein, refers to percent moisture of biomass. The moisture content is calculated as grams of water per gram of wet biomass (biomass dry matter plus water) times 100%.

The term "Ammonia Fiber Explosion" or "Ammonia Fiber Expansion" (hereinafter "AFEX") pretreatment" as used herein, refers to a process for pre-treating biomass with ammonia to solubilize lignin/hemicellulose and redeposit it from in between plant cell walls to the outer plant cell wall surfaces of the biomass. In some embodiments the ammonia employed is anhydrous liquid ammonia. In some embodiments the biomass used for AFEX pretreatment has reduced moisture. An AFEX pretreatment disrupts the lignocellulosic matrix, thus modifying the structure of lignin, partially hydrolyzing hemicellulose, and increasing the accessibility of cellulose and the remaining hemicellulose to subsequent enzymatic degradation. Lignin is the primary impediment to enzymatic hydrolysis of native biomass, and removal or transformation of lignin is a suspected mechanism of several of the leading pretreatment technologies, including AFEX. However in contrast to many other pretreatments, the lower temperatures and non-acidic conditions of the AFEX process prevent lignin and/or hemicellulose from being converted into furfural, hydroxymethyl furfural, phenolics and organic acids that could negatively affect enzyme/microbial activity. The process further expands and swells cellulose fibers and further breaks up amorphous hemicellulose in lignocellulosic biomass. These structural changes open up the plant cell wall structure enabling more efficient and complete conversion of lignocellulosic biomass to value-added products while preserving the nutrient value and composition of the material. See, for example, the methods described in U.S. Patent Nos. 6,106, 888, 7187,176, 5,037,663, and 4,600,590.

The term "bio-product" as used herein refers to products obtained from enzymatic hydrolysis and/or fermentation of pretreated biomass. Examples of bio-products include biofuels (e.g., one or more alcohols, including ethanol), hydrocarbons, and biochemical products (e.g., organic acids, such as lactic acid or succinic acid) produced from pretreated biomass.

The term "bio-product production unit" as used herein refers to that portion of a bio-product production process in a bio-product production facility in which pretreated lignocellulosic biomass is processed and converted into the bio-product.

The term "integrated bio-product production facility" as used herein refers to a bio-product production facility configured to produce a bio-product and a nutrient-containing byproduct from pretreated lignocellulosic biomass, and to further use solubles extracted from the pretreated lignocellulosic biomass for at least one of the following two purposes: (1) to promote or induce enzyme production by microorganisms, and (2) to stimulate growth for fermenting and/or enzyme-producing microorganism. An "integrated biofuel production facility" produces a biofuel in the above-described integrated manner and can also be referred to as an "integrated biorefinery."

The term "nutrient-containing byproduct" as used herein refers to a byproduct produced from production of bio-product from lignocellulosic biomass. The nutrient-containing byproduct can include food precursor components. Corn Steep Liquor (CSL) is one example of a nutrient-containing byproduct.

The term "diluted pretreated biomass hydrolysate" as used herein refers to a pretreated plant biomass hydrolysate containing no less than about 3% moisture. The diluted pretreated biomass hydrolysate can be a diluted ammonia pretreated biomass hydrolysate.

The term "Corn Steep Liquor" as used herein refers to a nutrient-containing by- product of corn wet-milling. CSL is a mixture of soluble protein, amino acids, carbohydrates, organic acids (e.g., lactic acid), vitamins and minerals.

The term "solubles" as used herein refers to components extracted from pretreated biomass using an appropriate solvent. The solubles can include at least one "stimulant" that is a microbial growth stimulant and an agent that induces enzyme production from a microbe. The biomass can be a lignocellulosic biomass. The solvent employed is water

### Biomass Conversion to Commercial Products

Nearly all forms of lignocellulosic biomass, i.e., plant biomass, such as monocots, comprise three primary chemical fractions: hemicellulose, cellulose, and lignin. Hemicellulose is a polymer of short, highly-branched chains of mostly five-carbon pentose sugars (xylose and arabinose), and to a lesser extent six-carbon hexose sugars (galactose, glucose and mannose). Dicots, on the other hand, have a high content of pectate and/or pectin, which is a polymer of alpha-linked glucuronic acid. Pectate may also be "decorated" with mannose or rhamnose sugars. These sugars are highly substituted with acetic acid.

Because of its branched structure, hemicellulose is amorphous and relatively easy to hydrolyze (breakdown or cleave) to its individual constituent sugars by enzyme or dilute acid treatment. Cellulose is a linear polymer of glucose sugars, much like starch, which is the primary substrate of corn grain in dry grain and wet mill ethanol plants. However, unlike starch, the glucose sugars of cellulose are strung together by β-glycosidic linkages which allow cellulose to form closely-associated linear chains. Because of the high degree of hydrogen bonding that can occur between cellulose chains, cellulose forms a rigid crystalline structure that is highly stable and much more resistant to hydrolysis by chemical or enzymatic attack than starch or hemicellulose polymers. Lignin, which is a polymer of phenolic molecules, provides structural integrity to plants, and remains as residual material after the sugars in plant biomass have been fermented to ethanol. Lignin is a by-product of alcohol production and is considered a premium quality solid fuel because of its zero sulfur content and heating value, which is near that of sub-bituminous coal.

Typical ranges of hemicellulose, cellulose, and lignin concentrations in plants are available at the website www1.eere.energy.gov/biomass/feedstock_databases.html. Typically, cellulose makes up 30 to 50% of residues from agricultural, municipal, and forestry sources. Cellulose is more difficult to hydrolyze than hemicellulose, but, once hydrolyzed, converts more efficiently into ethanol with glucose fermentation than hemicellulose. In contrast, the sugar polymers of hemicellulose are relatively easy to hydrolyze, but do not convert as efficiently as cellulose using standard fermentation strains (which produce ethanol from glucose). Although hemicellulose sugars represent the "low-hanging" fruit for conversion to ethanol, the substantially higher content of cellulose represents the greater potential for maximizing alcohol yields, such as ethanol, on a per ton basis of plant biomass.

Conventional methods used to convert biomass to alcohol include processes employing a concentrated acid hydrolysis pretreatment, a two-stage acid hydrolysis pretreatment as well as processes employing any known conventional pretreatment, such as hydrothermal or chemical pretreatments, followed by an enzymatic hydrolysis (i.e., enzyme-catalyzed hydrolysis) or simultaneous enzymatic hydrolysis and saccharification. Such pretreatment methods can include dilute acid hydrolysis, high pressure hot water-based methods, i.e., hydrothermal treatments such as steam explosion and aqueous hot water extraction, reactor systems (e.g., batch, continuous flow, counter-flow, flow-through, and the like), AFEX , ammonia recycled percolation (ARP), lime treatment and a pH-based treatment. However, pretreatment-hydrolysis of plant biomass can often result in the creation and release of other chemicals that inhibit microbial fermentation. These inhibitors (i.e. furfural) are largely the product of sugar degradation, and methods to remove these inhibitors or to reduce their formation or strains resistant to the inhibitors are needed.

Several of these methods generate nearly complete hydrolysis of the hemicellulose fraction to efficiently recover high yields of the soluble pentose sugars. However, chemical solubilization of hemicellulose also produces toxic products, such as furan derivatives, which can inhibit downstream microbial reactions (e.g., fermentation). Regardless, the hydrolysis of hemicellulose facilitates the physical removal of the surrounding hemicellulose and lignin, thus exposing the cellulose to later processing. However, most, if not all, pretreatment approaches do not significantly hydrolyze the cellulose fraction of biomass.

Biomass conversion to alcohol also poses unique fermentation considerations. The *Saccharomyces cerevisiae* yeast strains used in conventional corn ethanol plants for example, can ferment glucose, but cannot ferment pentose sugars such as xylose. Additionally, there is currently no naturally occurring microorganism that can effectively convert all the major sugars present in plant biomass to ethanol. Therefore, genetically engineered yeast or bacteria, which can, in theory, ferment both glucose and xylose to alcohol, is used for biomass to alcohol processes. However, in practice, co-fermentation is inefficient and glucose fermentation is still the main reaction for ethanol production.

### Description of the Embodiments

In one embodiment of the method described herein, an integrated bio-product production facility, such as a biofuel production facility, is configured to generate pretreated lignocellulosic biomass for forming both bio-product and enzymes useful in making bio-products. In one embodiment, this integrated approach is utilized to produce ethanol and the enzyme utilized in ethanol production without washing, detoxifying or adding supplemental (exogenous) nutrients to the pretreated biomass. As such, the pretreated lignocellulosic biomass is not only serving as a source of carbon and nitrogen, but also providing other nutrients for the biofuel production facility. In one embodiment, the process further includes yeast production. The lignocellulosic biomass is pretreated with ammonia, such as with an Ammonia Fiber Expansion (AFEX) treatment, to produce reactive, highly fermentable plant materials by reducing inhibitory degradation product generation and enriching the nitrogen content of the pretreated materials.

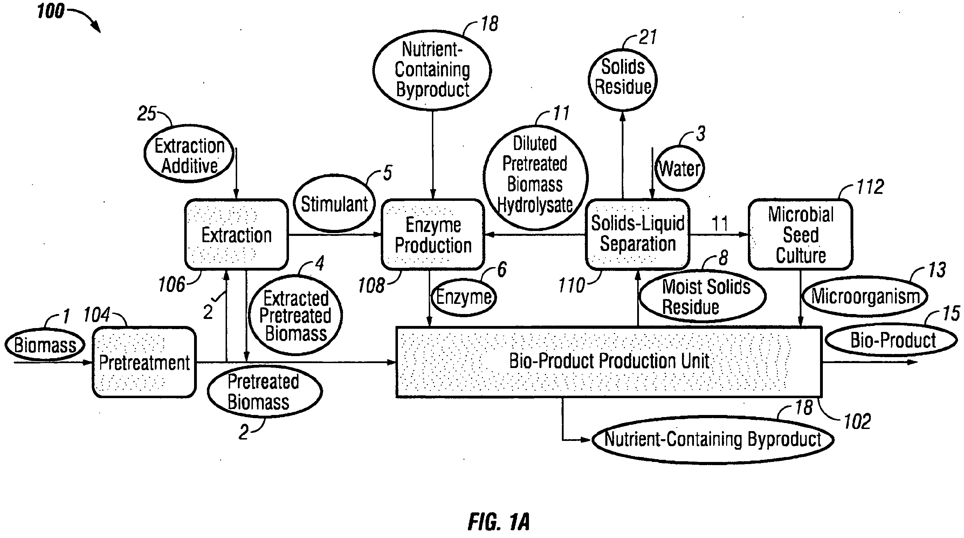

FIG. 1A shows one embodiment of a biofuel production facility 100 utilizing a bio-product production unit (BPU) 102 to produce a bio-product 15. In the embodiment shown in FIG. 1A, biomass 1 is provided to a pretreatment unit104 which pretreats the biomass 1 to produce pretreated biomass 2. In one embodiment, the pretreatment unit 104 is an AFEX treatment unit. A portion of the pretreated biomass 2 is provided to an extraction unit 106 to extract solubles from the pretreated biomass 2 using a suitable solvent (e.g., water), while the remainder of the pretreated biomass 2 is provided to the BPU 102. In one embodiment, between about 20% and about 40% of the pretreated biomass 2 is provided to the water extraction unit 106. In one embodiment, about 30% to about 35% is provided to the water extraction unit 106.

In one embodiment, the biomass 1 is lignocellulosic biomass (e.g., untreated corn stover). The biomass is derived from one or more plants. At least one of the one or more plants can be a monocot. The monocot can include corn stover, grass, corn stover, sorghum, sugarcane bagasse, wheat, rice, maize, or a combination thereof. The grass can include switchgrass, miscanthus, reed canary grass, or a combination thereof.

In one embodiment, the pretreated biomass 2 is AFEX-pretreated corn stover (AFEX-CS). As described above, the pretreated biomass 2 can undergo extraction within the extraction unit 106. In some embodiments, a portion of the pretreated biomass 2 is extracted. In other embodiments, a majority of the pretreated biomass 2 is extracted. For example, about 5% to about 70% of the biomass 2 can be extracted in the extraction unit. In some embodiments, about 20% to 50% of the biomass 2 can be extracted in the extraction unit. After extraction at least a portion of the extracted pre-treated biomass 4 can be sent to the bio-product production unit (BPU) 102.

The extraction unit 106 extracts at least one stimulant 5, which can be added to the enzyme production Unit 108. In one embodiment, the stimulant 5 is a water extract of the pretreated biomass 2.

The enzyme production unit 108 contains a microorganism that can secrete a cellulase and/or hemicellulase. In some embodiments, the enzyme-producing microorganism is *Trichoderma reesei.* In one embodiment, the enzyme-producing microorganism is *A. awamori.* In some embodiments, the enzyme-producing microorganism is a combination of microorganisms, one of which is *Trichoderma reesei.*

In one embodiment, the bio-product 15 is a biofuel, such as an alcohol and/or a hydrocarbon. In one embodiment, the bio-product 15 is a biochemical, such as an organic acid and succinic acid; the nutrient-containing byproduct 18 can also be CSL.

The pretreated biomass 2 is extracted in an extraction unit 106 with an extracting additive 25 to produce extracted solubles, i.e., a stimulant 5 (of microbial growth and of microbial enzyme production) and extracted pretreated biomass 4. Any suitable conditions configured to extract the solubles may be used. In one embodiment, the solubles are extracted with water as the extracting additive 25. In one embodiment, the solubles are extracted under alkaline conditions, such as with a pH between about 7 and 12, such as a pH between about 8 and 9.

The extracted pretreated biomass 4 is provided to the line containing the pretreated biomass 2; thereafter, the combined components (extracted pretreated biomass 4 and pretreated biomass 2) enter the BPU 102.

The extracted pretreated biomass 4 can have any suitable moisture content. In one embodiment, the moisture content is no greater than 95%. In some embodiments, the moisture content in the extracted pretreated biomass 4 is as low as 3%. In other embodiments, the moisture content in the extracted pretreated biomass 4 ranges from 5% to 75%, with ranges of 50% to 75% being quite common. The extracted pretreated biomass 4 has reduced carbohydrate content as compared to the pretreated biomass 2. In one embodiment, carbohydrate content in the extracted pretreated biomass 4 is reduced by up to 7% as compared to the pretreated biomass 2. In some embodiments, carbohydrate content is reduced by at least 7% up to 40%.

The stimulant 5 can have any suitable solids loading, i.e., any suitable amount of solid matter can be present. In one embodiment, a solids loading of up to 18% is achieved. In one embodiment, the solids loading may be higher, such as up to 50% solids loading. The stimulant 5 is then provided to an enzyme production unit 108 which is also provided with a nutrient-containing byproduct (i.e., enzyme-producing microorganism) 18 exiting the bio-product production unit 102. The resulting enzyme 6 is then providing to the bio-product production facility 102 for use in producing the bio-product 15. In one embodiment, the enzyme 6 is a cellulase and/or hemicellulase from *Trichoderma reesei (T. reesei)* fermentation. In one embodiment, the enzyme 6 is from *A. awamori.*

Moist solids residue 8 exiting the bio-product production unit 102 is separated in a solids-liquid separation unit 110 (optionally, with added water 3) to produce a diluted pretreated biomass hydrolysate 11 and a solids residue 21 containing less moisture than the moist solids residue 8. In one embodiment, the solids-liquid separation unit 110 comprises a screw press. The diluted pretreated biomass hydrolysate 11 can be provided to a microbial seed culture 112 and also to the enzyme production unit 108 for use as a sugar source for the nutrient-containing byproduct 18 (i.e., enzyme producing microorganism).

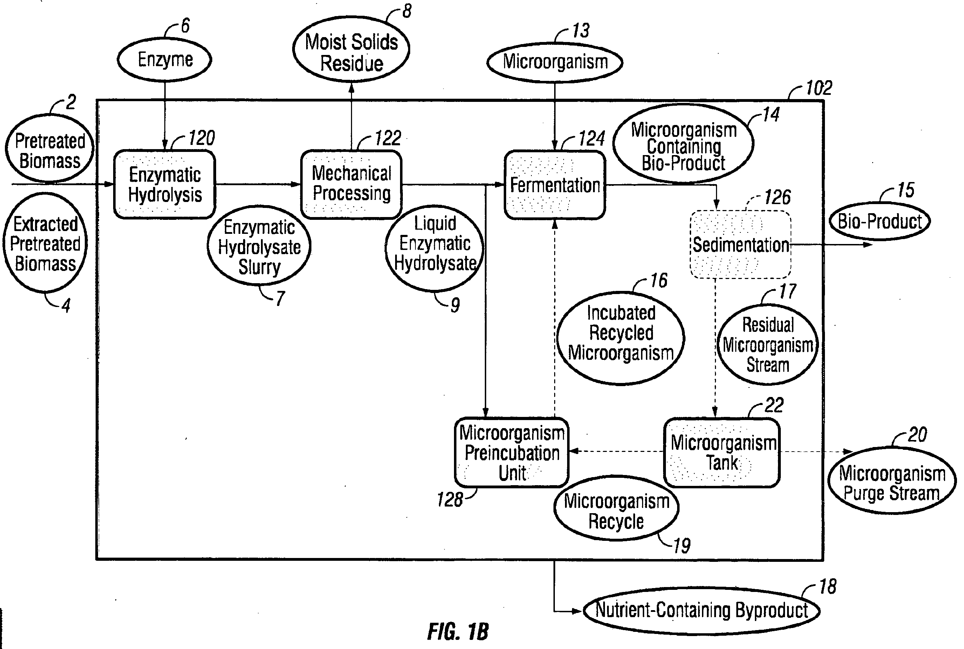

The microorganism 13 produced with the microbial seed culture 112 is provided to the BPU 102. Any suitable type of microorganism 13 can be used and/or produced. In one embodiment, fresh native and recombinant S. *cerevisiae* 424A(LNH-ST) is used and/or produced. Any suitable amount of microorganism 13 can be used and/or produced, with the amount varying depending on how much of the microorganism 13 is recycled with a microorganism recycle stream 19 shown in FIG. 1B and how much exits in the microorganism purge stream 20.

As shown in FIG. 1B, the BPU 102 comprises an enzymatic hydrolysis unit 102 to which the enzyme 6 is provided along with the pretreated biomass 2 and the extracted pretreated biomass 4. After undergoing enzymatic hydrolysis 120, an enzymatic hydrolysate slurry 7 is produced which is provided to a mechanical processing unit 12, such as a suitable type of press, to produce the residual solids 8 and liquid enzymatic hydrolysate 9. The liquid enzymatic hydrolysate 9 is provided to both the fermentation unit 124 into which the microorganism13 is also provided, and to a microorganism preincubation unit 128.

The portion of the liquid enzymatic hydrolysate 9 which enters the fermentation unit 124 (along with the microorganism 13) produces a microorganism-containing bio-product 14 which can optionally be provided to a sedimentation unit 126 to produce the bio-product 15. In embodiments which include the sedimentation unit 126, residual microorganisms 17 can also exit the sedimentation unit 126. Any residual microorganisms 17 are provided to a microorganism tank 22 where the output is split into the microorganism recycle stream 19 and the microorganism purge 20. Varying amounts of the microorganism 17 can be purged, for example, after each recycling event. For example, 1% to 40% of the microorganism 17 can be purged after each recycle event. In some embodiments, 10% of the microorganism 17 can be purged.

Additionally, a cell preincubation unit 128 is configured to receive the microorganism recycle stream 19 from the microorganism tank 22 as well as liquid enzymatic hydrolysate 9 exiting the mechanical processing unit 122. The cell preincubation unit 128 preincubates the microbe in the microorganism recycle stream 19 to produce an incubated recycled microorganism 16 which is provided to the fermentation unit 124. In one embodiment, the microbe is yeast, fungi and/or bacteria.

In one embodiment, provided for exemplary purposes only, a bio-product production facility 102 can operate as follows: On a basis of 1000 g untreated corn stover, 1020 g of AFEX-pretreated corn stover (AFEX-CS) is produced. Approximately 1/3 of the AFEX-CS is extracted at 18% w/w solids loading to yield water-extracted stimulant 5 that can be used for enzyme induction/production. Enzymes that can be induced include a cellulase and/or hemicellulase from *Trichoderma reesei.* The soluble sugars in the pressed residual solids are further diluted to generate a sugar stream for seed culture preparations for *Trichoderma reesei* RUT-C30 and *Saccharomyces cerevisiae.* Taking into account the carbohydrate streams used for preculture preparation and induction, 10% of the carbohydrate in the input untreated corn stover is used for enzyme production. The extracted AFEX-pretreated corn stover, in which the total carbohydrate is reduced by 7%, is fed to the pretreated AFEX-CS stream, and the combined stream is enzymatically-hydrolyzed (e.g., using cellulase and/or hemicellulase enzyme(s), some of which can be from *Trichoderma reesei).* After four days of enzymatic hydrolysis, the enzymatic hydrolysate slurry is separated to yield a liquid enzymatic hydrolysate and moist residual solids by mechanical processing means, such as a pneumatic press. The liquid enzymatic hydrolysate contains sugars that are fermented to ethanol using the two stage ethanol fermentation. The fermentation can employ fresh native and/or recombinant S. *cerevisiae* 424A(LNH-ST) (e.g., 0.05 g/L of fresh native S. *cerevisiae* for glucose fermentation and 1.0 g dry wt/L of fresh S. cerevisiae 424A(LNH-ST) inoculum for xylose fermentation). Yeast from a recycle stream can also be added or employed. During fermentation native yeast cells can be produced at a yield of 20.8 g dry yeast cells/1 kg untreated corn stover. 10% of the recombinant S. *cerevisiae* 424A(LNH-ST) can be purged after each recycle event.

In one embodiment, the nutrient content (protein, vitamins and trace elements) of an AFEX-treated enzymatic hydrolysate, such as AFEX-treated corn stover (AFEX-CS) enzymatic hydrolysate, has a solids loading of up to 18% with at least 85% of the carbohydrate solubilized. In one embodiment, a method for supporting both ethanol and in-house enzyme production using AFEX-CS is provided. (See also Example 6, which provides an economic model for the exemplary process).

In contrast to conventional practice, the embodiments described herein do not need to utilize added nutrients to support microbial growth for fermentation. In one embodiment, nutrients present in biomass, such as cellulosic biomass, are solubilized in a liquid extract of the hydrolysate generated by AFEX pretreatment and used to stimulate microbial growth and enzyme synthesis. In one embodiment, AFEX-treated biomass can support fermentation activities beyond ethanol production due to the presence of excess minerals.

By reducing or eliminating the use of added nutrients and enzymes, and, in some embodiments, yeast, the processes described herein are more economical than conventional methods.

In one embodiment, in-house enzyme production reduces the exogenous enzyme requirement from 10 mg/g biomass down to no more than 1 mg/g biomass. The overall cost of enzyme reduced primarily due to the ability to utilize sugars (both monomers and oligomers) from AFEX-CS for enzyme production.

Surprisingly, water soluble extractives isolated from pretreated biomass, such as AFEX pretreated lignocellulosic biomass provide a potent inducer for production of cellulases and hemicellulases. Further improvements to industrial fungal strains to optimize utilization of pretreated lignocellulosic biomass as the sole carbon source for production of biomass degrading enzymes might greatly reduce the cost of cellulosic biorefineries.

In one embodiment, enzyme cost is further reduced by at least partial recycling of the enzymes, such as with countercurrent contacting of spent biomass hydrolysate with fresh, unhydrolysed solids. Reducing the total amount of hydrolytic enzymes (from 15 mg protein/gm stover) also reduces the cost of enzyme. Use of an in-house enzyme production approach takes advantage of the abundant cellulosic biomass as the source of carbohydrate and minerals, thus eliminating the requirement for expensive substrates such as lactose or sopharose while achieving superior enzyme induction.

The enzyme titer produced by "in-house" stimulation of enzyme production, is sufficient to support high solids loading (at least 18% w/w solids loading) with enzymatic hydrolysis at an enzyme loading of 15 mg protein/g corn stover. As such, in various embodiments, and in contrast to conventional methods, the enzyme mixture is not further concentrated, no protein stabilizer is added and/or no preservatives are added.

In one embodiment, high cell density fermentation can be conducted without the need for significant fresh cell inoculum due to the high recyclability of yeast cells in the hydrolysate. Thus, native yeast from a first stage ethanol fermentation is one example of a useful co-product that can be recycled into the fermentation process or sold. Therefore, the proposed biorefinery approach creates a scenario in which the processes described herein can enhance the production of growth stimulants, enzyme production stimulants, food precursors, and yeast to thereby stimulate growth of the biofuels industry. These products can be recycled back into an appropriate step of the process (see FIG. 1A) or sold to generate revenue. Besides food and animal feed industries, yeast is useful for generating specialty products including invertase, beta-glucans, phospholipids and ergosterol. Thus, yeast cells are a valuable product of the processes described herein.

In one embodiment, native yeast serves as an agent to capture and concentrate the nutrients from plant biomass while simultaneously producing ethanol as fuel. However, in some embodiments a solid-liquid separation precedes fermentation to facilitate yeast cell separation from broth. Such bioconversions can therefore be completed in the Separate Hydrolysis and Fermentation (SHF) mode, rather than the Simultaneous Saccharification and Co-fermentation (SSCF) mode.

In one embodiment hydrolysate from AFEX-treated biomass, such as AFEX-CS, especially at high solids loading, is nutrient-rich and highly fermentable, containing a nutrient level similar to malt wort for beer production. This is in contrast to conventional practices which are based on the perception that an enzymatic hydrolysate from biomass must be preceded by detoxification and nutrient supplementation to improve its general fermentability. This perception may be due to the nature of acidic pretreatment in which detoxification (such as over-liming) and/or washing of pretreated materials to remove inhibitory compounds renders the pretreated biomass nutrient-deficient.

In one embodiment, a biofuel production facility is provided comprising a two-stage integrated alcohol (e.g., ethanol) fermentation and a cellulase and/or hemicellulase enzyme fermentation with a suitable microorganism (e.g., *Trichoderma reesei* RUT-C30) using AFEX-pretreated biomass (e.g., cellulosic biomass such as corn stover, switchgrass, rice straw, and the like) as a source for carbohydrate and minerals.

Embodiments of the invention will be further described by reference to the following examples, which are offered to further illustrate various embodiments of the present invention.

### EXAMPLE 1

The feasibility of extracting proteins from switchgrass harvested in the spring while simultaneously producing sugars through enzymatic hydrolysis was examined. Conditions for solid/liquid extraction using aqueous ammonia were optimized and compared to other solvents. Potential process flow schemes were examined with respect to their sugar and protein yields before a complete material balance of the final process was determined. The solution after removal of some of the proteins and ammonia is the MGS.

### Materials and Methods

### Feedstock

The feedstock used in this experiment was Alamo Switchgrass (Switchgrass or GS) obtained from Auburn University and harvested on May 22, 2005. The moisture content of the material was approximately 9%. All material was ground to less than 2 mm prior to testing.

### Pretreatment

The AFEX pretreatment was performed in a 300 mL stainless steel pressure vessel. Water was mixed with the switchgrass to increase the moisture content to 80% dry weight basis. Glass spheres were added to minimize void space, thereby reducing the amount of ammonia in the gaseous state. The lid was bolted shut, and a sample cylinder loaded with 1 (+/-0.04) g NH₃ per g dry biomass, allowing the ammonia to be charged into the vessel. The reactor was heated using a 400W PARR heating mantle, and allowed to stand at 100°C (+/- 1°C) for five minutes. The pressure was explosively released by rapidly turning the exhaust valve. The treated samples were removed and were placed in a fume hood overnight to remove residual ammonia.

### Hydrolysis

The enzymatic hydrolysis procedure was based upon the 2004 LAP-009 protocol entitled, *Chemical Analysis and Testing (CAT) Standard Procedures,* from the National Renewable Energy Laboratory (NREL). Samples were hydrolyzed in Erlenmeyer flasks at 10% solid loading buffered to pH 4.8 by 1 M citrate buffer. Spezyme CP (Genencor; Palo Alto, California) cellulase was loaded at 15 FPU/g glucan (31 mg protein/g), and β-glucosidase (NOVOZYME 188; Bagsvaerd, Denmark) at 64 pNPGU/g glucan. All samples were incubated at 50°C with 200 rpm rotation. Sugar concentration after 168 hours was determined using a Waters High Performance Liquid Chromatograph (HPLC) system equipped with a Bio-Rad (Richmond, California) Aminex HPX-87P carbohydrate analysis column. Degassed HPLC water with a flow rate of 0.6 mL/min was used as the mobile phase, while the temperature in the column was kept constant at 85°C.

### Protein Extractions

Screening for optimal protein extraction conditions was performed using a Dionex (Sunnyvale, California) ASE 200 Accelerated Solvent Extractor. Extractions were performed in duplicate, using two separate extractions per sample, at 50°C, with 3% ammonium hydroxide, at pH = 10.5, after an AFEX treatment using 11:1 liquid/solid ratio. Use of 1500 psi (∼102 atm) for the extractions reduced residence time from 30 minutes down to 3 minutes. For experiments involving varying the pH, hydrochloric acid was used to reduce the pH. The pH of the solution was measured after the extraction was complete. Once the optimal extraction conditions were obtained, all further extractions were performed in flasks for 30 minutes with a 10:1 liquid/solid ratio while continuously stirred.

Due to the presence of ammonia nitrogen, both during the AFEX pretreatment and subsequent extractions, it was not possible to use standard nitrogen analysis methods (the Kjehldahl or Dumas methods) to measure total protein content. Instead, protein concentration was measured using a Pierce (Rockford, Illinois) bichronimic acid colorimetric assay kit using bovine serum albumin (BSA) as a standard. To reduce the effects of interfering agents, such as ammonium salts, lignin components, and glucose, the proteins were first precipitated and resolubilized (20). A 100 µL 0.15% sodium deoxycholate was added to 100 µL protein solution and allowed to sit for 15 minutes. 200 µL of 15% trichloroacetic acid solution was added, and allowed to sit at 2°C overnight. The mixture was centrifuged at 13,000 RPM for 10 minutes, and the resulting pellet washed with acetone. The pellet was resolubilized in a buffer solution containing 0.1 M Tris, 2.5M urea, and 4% SDS. Known concentrations of protein extracts were used to calibrate the protein recovery of this method.

### Composition Analysis

The weight and moisture content of the remaining solid fraction after each processing step was measured for determining the mass balance in the system. The composition of each of these fractions was determined based upon NREL's LAP 002 protocol. Ash content was determined by heating 1.5 g of biomass at 575°C for 24 hours and measuring the weight loss. Water and ethanol extractives were removed using a soxhlet extraction. A portion of the extracted biomass was digested in concentrated (72%) sulfuric acid in a 10:1 liquid: solid ratio at 30°C for one hour. The solution was diluted to 4% sulfuric and autoclaved at 120°C for one hour, and then analyzed for sugar components using a Bio-Rad (Richmond, California) Aminex HPX-87H HPLC column using sulfuric acid as the mobile phase. The acid insoluble lignin was measured as the remaining solid after hydrolysis less the ash content in the solid residue.

### Results and Discussion

### Composition

The composition of approximately 80% of the mass of the Alamo switchgrass used in this example is shown in Table 1 below. The remaining mass is primarily water soluble components, such as minor organic acids, and acid soluble lignin.

**Table 1: Acid Insoluble (AI) Composition of Alamo (g/100g dry matter) Switchgrass**

| Component | % Value |
|---|---|
| Glucan | 26.4 |
| Xylan | 16.4 |
| Arabinan | 3.5 |
| Sucrose | 3.4 |
| Protein | 7.3 |
| Al Lignin | 10.8 |
| Lipids | 7.3 |
| Ash | 4.8 |
| Total | 79.9 |

As Table 1 shows, the amount of protein was lower than reported in literature for other strains of switchgrass. Switchgrass grown as a biomass energy crop and harvested early in the growing season would likely have protein contents closer to 10%, which may be more suitable for integrated protein and sugar processing. The amount of fiber present was also lower as compared to switchgrass harvested at a later date, which seems to suggest lower sugar yields would also result from using an earlier cut. However, early cut switchgrass is less recalcitrant than that harvested in the fall. As such, a lower cellulose and hemicellulose content may not be a significant factor. The low amount of lignin may imply less interference with hydrolysis, as well as fewer harmful degradation products, which could inhibit sugar production or otherwise be present in the protein product. The ash content was higher as compared to ash content in switchgrass harvested later in the season, i.e., close to full maturity of the plant.

The essential amino acid profile for the Alamo switchgrass used in this study together with literature values for corn grain and soybean (Parkinson et al., Aquaculture 186: 293-310 (1999)) is shown in Table 2 below:

**Table 2: Essential Amino Acid Profile for Alamo Switchgrass (SG), Soy and Corn**

| | Arg | His | Ile | Leu | Lys | Met | Phe | Thr | Val |
|---|---|---|---|---|---|---|---|---|---|
| SG | 2.1 | 1.8 | 3.7 | 5.6 | 7.4 | 0.6 | 9.1 | 4.9 | 6.1 |
| Soy | 7.5 | 2.6 | 4.9 | 7.7 | 6.1 | 1.6 | 5.1 | 4.3 | 5.1 |
| Corn | 2.9 | 1.6 | 4.3 | 16.2 | 1.6 | 2.3 | 5.9 | 3.1 | 4.4 |

From left to right in Table 2, the amino acids include: Arginine (Arg), Histidine (His), Isoleucine (Ile), Leucine (Leu), Lysine (Lys), Methionine (Met), Phenylalanine (Phe), Threonine (Thr) and Valine (Val). As Table 2 shows, the switchgrass was relatively high in lysine. As an essential amino acid, the amount of lysine is often the first limiting amino acid in poultry diets. High values for phenylalanine and valine are also present in the switchgrass. While the switchgrass is lower in leucine, arginine, and methionine amounts, these amino acids are relatively abundant in corn. Thus, a corn-switchgrass protein diet would balance out these deficiencies, and thus may provide an alternative to a corn-soy diet.

### Extraction Optimization

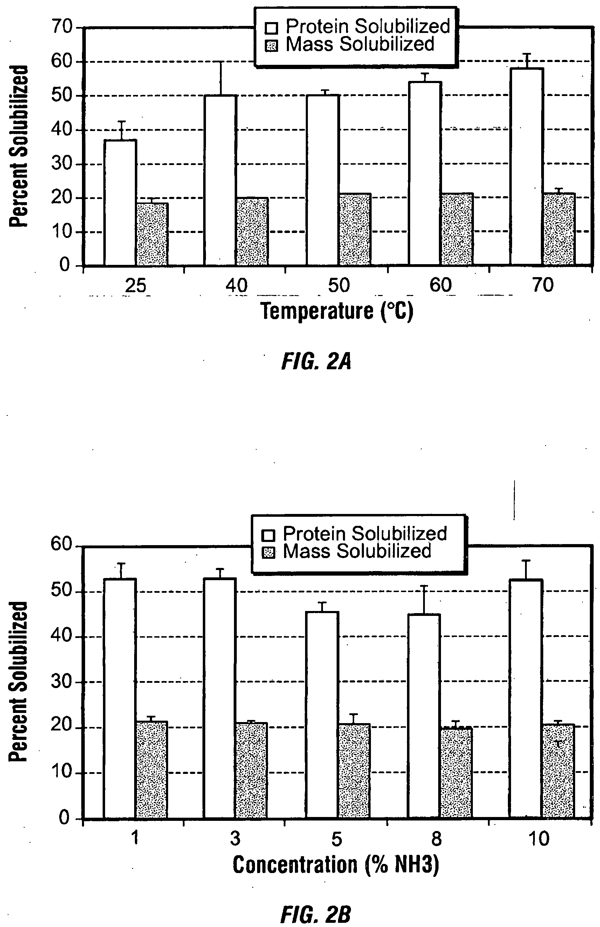

FIG. 2A shows the effect of extraction temperature on the overall protein and mass yields for AFEX treated switchgrass, with error bars representing the maximum and minimum values. Protein yields increased significantly from 25°C to 40°C, but further increases in temperature did not result in major improvements in protein yield. It is likely that most, if not all, of the proteins present in the switchgrass were in their natural state, with the harvesting and drying conditions utilized likely having had little to no impact. As such, the mild temperatures likely did not unfold the proteins or significantly affect their solubility.

FIG.2B shows the effect of ammonia concentration on protein and mass yields for the AFEX treated switchgrass. The protein yield remained constant as the levels of NH4⁺ were increased from 1% up to 3% by volume, but then began to drop off. This was most likely due to "salting out" of the protein as the protein's increase in salt concentration decreased the amount of water available to solubilize the protein. There did not appear to be any salting in effect, likely because 1% by volume salt solution was already a sufficient concentration to solubilize the protein. As FIG. 2B shows, the total mass solubilized was unaffected by salt concentration.

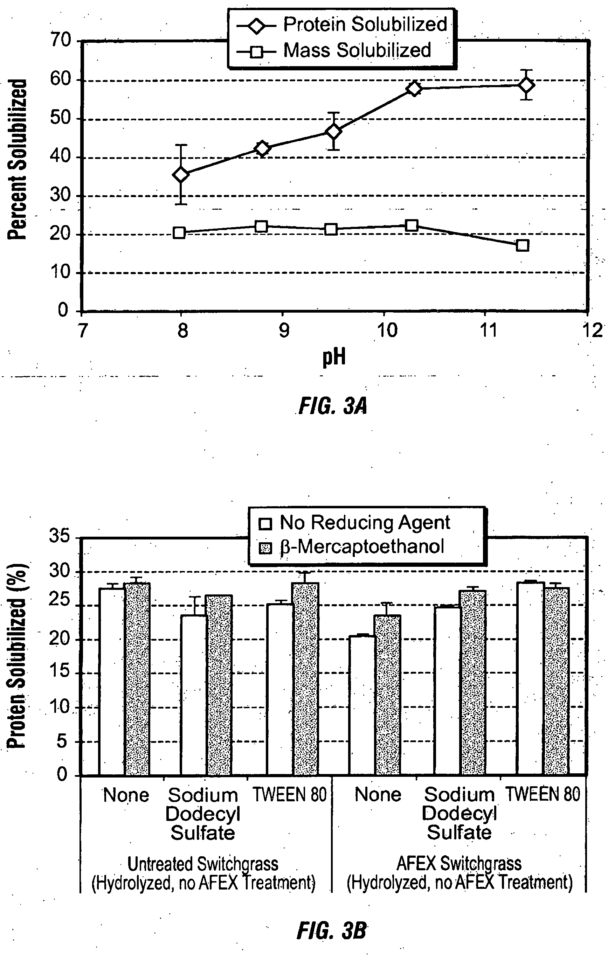

FIG. 3A shows the effect of pH on protein and mass yields, with the amount of protein extracted increasing dramatically from a pH of 8 to 10.5, before leveling off. As such, this testing suggests that the pH of the system may be a significant factor in determining protein yields. Most proteins have an acidic isoelectric point, the pH at which the protein will have no net charge, and therefore, be the least soluble in a polar medium. Thus, increasing the pH should increase protein solubility, as demonstrated herein. As FIG. 3A shows, the most alkaline solution also produced a significant drop in the total mass solubilized. As such, with less biomass in solution, it may be easier to purify the proteins. In addition, any biomass lost during extraction likely included hemicellulose, which can be hydrolyzed into sugars for ethanol production. Further increases in pH could be accomplished with a stronger base than ammonia. However, such an approach is likely to degrade the protein.

Attempts were made to improve yields through addition of a nonionic surfactant, namely TWEEN 80, a non-ionic surfactant, sodium dodecyl sulfate (SDS) and β-mercaptoethanol, a reducing agent. FIG. 3B is a graph showing the effect of reducing agents on protein yields for untreated switchgrass (hydrolyzed and no AFEX treatment) and AFEX treated switchgrass (hydrolyzed and AFEX treatment), with error bars representing the maximum and minimum values. No significant improvements were seen by addition of either surfactant or the reducing agent for the untreated switchgrass. However, addition of either TWEEN 80 or β-mercaptoethanol to AFEX treated switchgrass did increase protein removal. This result suggests that the AFEX process affects the proteins in some manner. It is possible this effect may be through the creation of sulfur-sulfur bonds cleaved by β-mercaptoethanol, or by proteins unfolding and exposing hydrophobic sites, which can be resolubilized with surfactants. The total mass of the switchgrass solubilized also increased with the addition of the surfactants, most likely due to interactions between the surfactants and hydrophobic portions of the switchgrass.

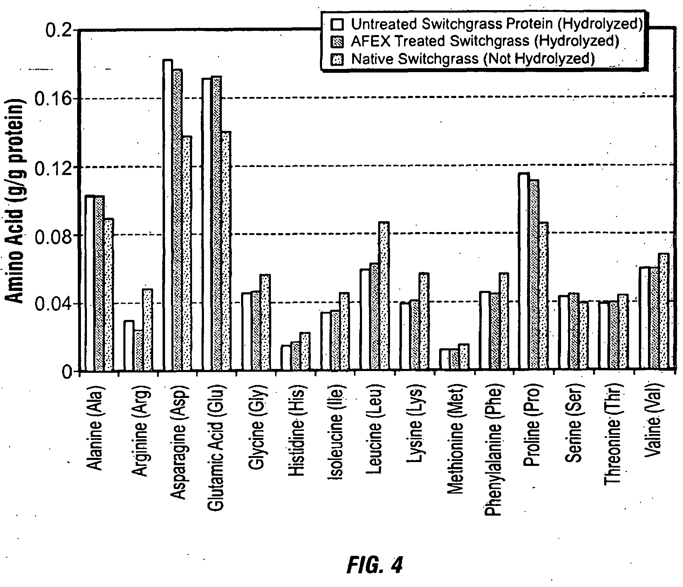

To determine whether AFEX pretreatment affects the types of proteins recovered, the composition of individual amino acids was determined. FIG. 4 is a graph showing amino acid profiles for untreated switchgrass protein, AFEX treated switchgrass protein, and native switchgrass protein and native switchgrass (not hydrolyzed and no AFEX treatment). Both the untreated and AFEX treated switchgrass were extracted at the optimal ammonia conditions without adding surfactant or reducing agent. Although the amino acid profile for the proteins solubilized during extraction compared to the total protein in the native switchgrass was quite different, the profile between extractions from untreated and AFEX treated grass exhibited only minor differences. As such, although AFEX does disrupt the cellular structure of the switchgrass, AFEX does not appear to release other proteins. As such, it is likely that the structure of the protein itself may affect protein recovery, rather than the structure of the plant being used as the biomass.

Thus, optimal extraction conditions for switchgrass were approximately 3% aqueous ammonia at a pH of 10 and temperature of 40-50°C. Total protein yields were approximately 40% by weight. However, AFEX did not appear, at least with this particular set of test conditions, to significantly improve yields of protein.

### EXAMPLE 2

This Example describes integration of sugar and protein recovery by performing extraction immediately after AFEX or performing extraction immediately after hydrolysis. Unless otherwise noted, all percentages are on a weight basis.

### Extraction Prior to Hydrolysis

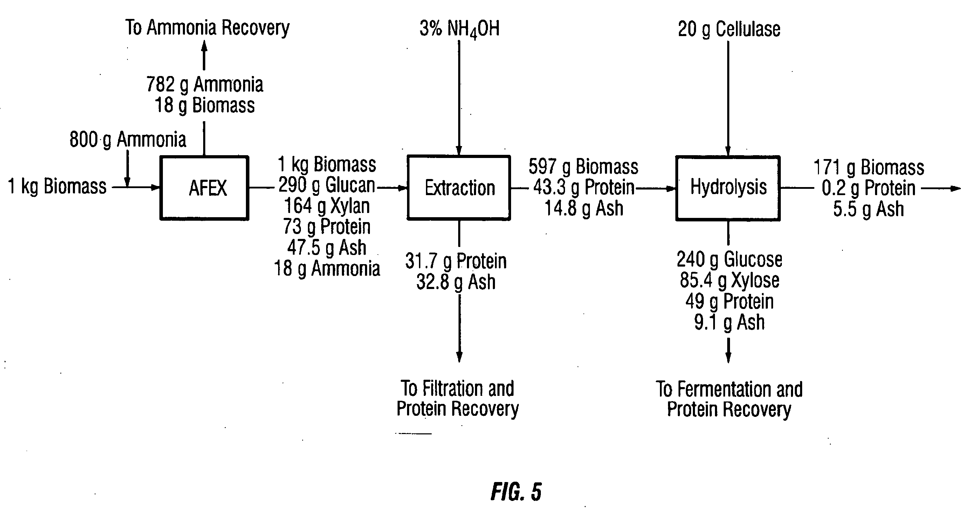

The overall mass balance for integrated sugar and protein with extraction prior to hydrolysis is shown in FIG. 5. Balances around the protein and ash content are given, as well as total mass and the amount of glucose and xylose produced. Final yields were 240 g glucose, 85.4 g xylose, and 80.7 g protein per kg dry biomass. Sugar recovery was approximately 74% by weight of theoretical values, indicating further improvements in sugar recovery can be made. Approximately 40% by weight of the protein was found in the extract and 60% by weight in the hydrolysate, demonstrating that protein must be recovered from both streams in order to be economical. Insoluble biomass was washed after hydrolysis to ensure all soluble components were recovered. The washing process may have acted as a second extraction to remove remaining proteins bound to insoluble portions of the biomass. Total protein yield was approximately 87% by weight of the total, taking into account both the switchgrass protein and the enzymes used in hydrolysis. However, no insoluble protein remained in the biomass, thus suggesting that the remaining protein was broken down and lost at some point during the process.

Approximately 40% by weight of the biomass was solubilized during the initial protein extraction step. The soluble fraction of the biomass after the proteins have been removed can be used as a Microbial Growth Stimulant (MGS). The protein can be concentrated and removed through ultrafiltration or heat precipitation, while the remaining solution can undergo further processing to provide the MGS.

Most of the ash was removed from the biomass during the first extraction step. Removal of the ash results in a final insoluble residue which can be burned to provide heat and power for the biofuel production facility. In this testing, only about 3% by weight of ash remained. As such, most of the ash was removable during a single step, which provides an economical means for producing a low ash content product.

Throughout this testing, approximately 17% by weight of the switchgrass remained insoluble. The residue is comprised primarily of unhydrolyzed fiber and insoluble lignin, with little to no protein or ash present. As such, this product would be useful as a source for heat and power in the biofuel production facility, thus reducing natural gas or coal requirements. Additionally, the lack of protein and ash would reduce the presence of NOx formation and slagging, respectively.

### Hydrolysis Prior to Extraction

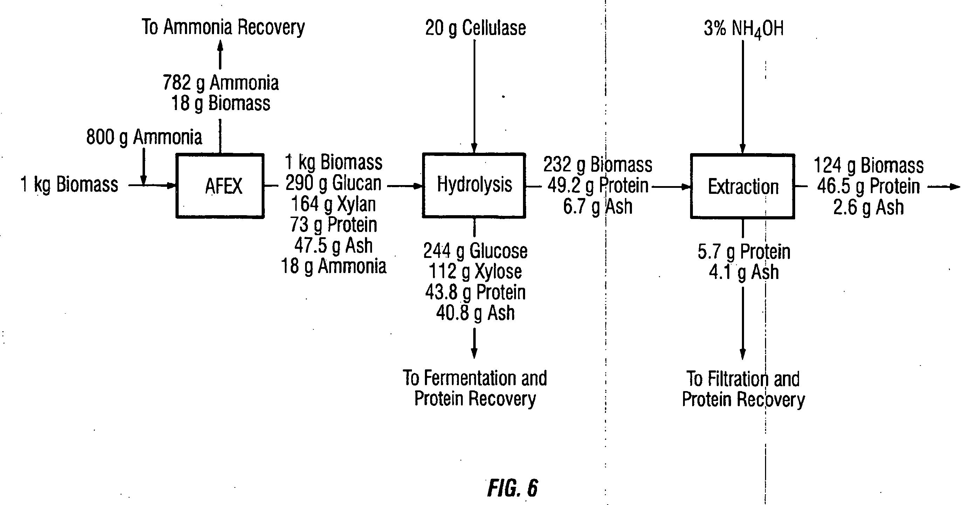

A separate process, focusing on performing hydrolysis prior to extraction, is shown in FIG. 6, with the amounts of reactants and products. Balances around the protein and ash content are given, as well as total mass and the amount of glucose and xylose produced. Here, sugar yields were slightly higher, with a total of 356 g compared to 325 g per kg biomass using the previous approach shown in FIG. 5. This result was mainly due to xylan conversion, indicating that xylan oligomers were likely extracted along with protein during the initial extraction step in the previous scenario. However, although approximately 60% by weight of the protein in the switchgrass was solubilized during hydrolysis, very little was extracted afterwards. It may be that during hydrolysis, other compounds were produced that interfered with the colorimetric analysis, thus increasing the error involved. This mass balance, however, relied solely on the individual amino acids rather than a colorimetric response, thus providing a more accurate representation of actual protein levels. Subsequent extractions on the final residue did not release more than a small fraction of the residual proteins, making it unlikely that further treatments can remove the residual protein.

The amount of insoluble material remaining was less than the amount which occurred when extraction was performed prior to hydrolysis (FIG. 5). Regardless, a sizeable amount of protein remained. Protein has lower energy content than lignin and its combustion generates NOx. Thus, an extraction prior to hydrolysis yields higher power yields, with slightly lower sugar yields.

### EXAMPLE 3

In this Example, an option for integrating sugar and protein recovery was studied by extraction prior to performing AFEX. However, sugar yields were much lower than the tests described in Example 2. Although not wishing to be bound by this proposed theory, it is possible that extracting proteins and other materials prior to AFEX changes the effects of AFEX pretreatment. AFEX also produces some organic acids that may inhibit hydrolysis, and, again, while not definite, it is possible that a prior extraction may produce more of these inhibitory acids. As such, washing of the switchgrass after AFEX treatment increased the sugar yields to approximately the same level as hydrolysis without any previous extraction.

### EXAMPLE 4

Corn Stover (CS) was obtained from the National Renewable Energy Laboratory (NREL). The CS contained 33.2% cellulose, 22.4% xylan, 3.3% arabinan and 2.3% protein as determined by the National Renewable Energy Laboratory (NREL) compositional analysis method as described in the NREL Standard Biomass Analytical Procedures; U.S. Department of Energy, 2008 and by the method described in S.P.S. Chundawat, R. Vismeh, L. Sharma, J. Humpula, L. Sousa, C.K. Chambliss, A.D. Jones, V. Balan, B.E. Dale, Multifaceted characterization of cell wall decomposition products formed during ammonia fiber expansion (AFEX) and dilute-acid based pretreatments, Biores Technol, 101 (2010) 8429-8438.

Rice straw (RS) was produced according to the method described in Zhong, C., Lau, M.W., Balan, V., Dale, B.E. & Yuan, Y.J. Optimization of enzymatic hydrolysis and ethanol fermentation from AFEX-treated rice straw. Appl. Microbiol. Biotechnol. 84, 667-676 (2009). The RS contained 4.7% cellulose, 15.1% xylan and 2.2% arabinan, as determined by the NREL methodology described above.

Both the CS and the RS were subjected to an AFEX treatment according to the method described above to produce AFEX-treated CS (AFEX-CS) and AFEX-treated RS (AFEX-RS), respectively. The AFEX treatment process utilized is also described in Zhong, C., Lau, M.W., Balan, V., Dale, B.E. & Yuan, Y.J. Optimization of enzymatic hydrolysis and ethanol fermentation from AFEX-treated rice straw. Appl. Microbiol. Biotechnol. 84, 667-676 (2009) and M.W. Lau, B.E. Dale, Cellulosic ethanol production from AFEX-treated corn stover using Saccharomyces cerevisiae 424A(LNH-ST), Proceedings of the National Academy of Sciences of the United States of America, 106 (2009) 1368-1373.

In this testing, solubles were extracted using water and an aqueous extract was used in a growth medium for the cellulase enzyme-producing fungus *T. reesei.*

### 18% SLE water extract preparation

AFEX-pretreated corn stover (AFEX-CS) was extracted with distilled water at a ratio of 1 g dry AFEX-CS to 4.6 g of water to produce an aqueous extract (18% solids loading equivalent). In each batch of washing, distilled water was preheated to 60-70°C and added to 100 g (dry weight equivalent) of AFEX-CS. The water content of the wetted AFEX-CS was reduced by pressing. The washing was conducted in three cycles, i.e. water-extract from a previous cycle of washing was used for the next cycle of washing. In the final cycle of washing, the moisture content of the extracted AFEX-CS was reduced to 77±3%. The AFEX-CS water extract was used for the fermentation. The preparation steps were as described in Lau et al, Biotechnol. Biofuels 2: 30 (2009). The total sugar solubilized was calculated by multiplying total soluble sugar in the water extract with total volume of the water extract from a given mass of dry AFEX-CS.

### 20% w/w CSL preparation

FermGold™ Corn Steep Liquor (CSL) (Lot: 154-07) from Cargill, Inc (Minneapolis, MN) was used as the protein supplement for fermentations. To prepare 20%w/w CSL, 200 g of FermGold™ CSL was diluted to total volume of 1 liter with distilled water after pH was adjusted to 5 with reagent grade KOH. The insoluble solids were separated from the liquid by centrifugation at 5,000 × g for 30 min. The 20% w/w CSL was sterile-filtered (0.22µm) and used for media preparation.

### Seed culture preparation

Media: 2% w/w corn steep liquor+20 g/L glucose +50mM phosphate buffer (adjusted to pH 5.5); and culture condition: 30°C, 200rpm agitation, 48 hr incubation time.

### Trichoderma Fermentation

Media : 60% v/v of seed culture and 5.5% solids loading equivalent of AFEX-CS wash stream+ 0.5 g of AFEX-CS in 50mL total volume; and culture condition: pH 5.5 (adjusted every 24 hr), 30°C, 200rpm agitation, 96 hr incubation time.

### Enzymatic Hydrolysis

Solids loading: 1% cellulose loading equivalent of AFEX-CS; hydrolysis mixture: 1:6 diluted of *Trichoderma* fermentation broth with varying loading of *Accellerase;* and hydrolysis condition: pH 4.8, 50°C, 250rpm agitation, 24 hr incubation time.

### Nutrient Content Analysis

### Ammonia

Free ammonia in AFEX-CS hydrolysate was analyzed by enzymatic assay (R-biopharm AG (Cat no: 11112732035, Darmstadt, Germany). The solution was diluted to an appropriate level for assay detection. The NADH reduction level, which indicates the concentration of ammonia in the solution, was measured as the absorbance at 340 nm wavelength using a spectrophotometer. A standard ammonia solution (control experiment) was tested to ensure the accuracy of the results. Other experimental details and enzymatic chemistry explanation are given by the manufacturer.

### Protein

The analyses for amino acid concentrations in AFEX-CS hydrolysate were conducted in the MSU Macromolecular Structure Facility using a High Performance Liquid Chromatography (HPLC) system equipped with a Nova Pak C18 (3.9mm×150mm; Waters). Operational details of the system were as described in Bergman, T. Carlquist, M., Advanced Methods in Protein Microsequence Analysts - Amino Acid Analysis by High Performance Liquid Chromatography of Phenylthiocarbamyl Derivatives, pp. 45-55 (1986). The amino acids involved in the analysis are Asparagine (Asp), Glutamic Acid (Glu), Serine (Ser), Glycine (Gly), Histidine (His), Theronine (Thr), Arginine (Arg), Alanine (Ala), Proline (Pro), Threonine (Thr), Valine (Val), Methionine (Met), Isoleucine (Ile), Lysine (Lys) and Phenylalanine (Phe).

### Determination of Protein Concentration in Complex Enzymes

The protein concentrations of commercial enzymes Accelerase 1000, Spezyme CP, Novozyme 188, Multifect Xylanase, and Multifect Pectinase were determined through nitrogen content analyses of the protein precipitate. Each complex enzyme was centrifuged (13,000 x g) for 5 min, and 0.20 mL of clear supernatant of the enzyme was combined with 0.25 mL 100% w/v trichloroacetic acid (TCA) and 0.80 mL distilled water to precipitate the protein in the enzyme solution. After 5 minute of incubation at 4°C, the mixture was centrifuged at 13,000 x g for 5 min and the supernatant was decanted. The precipitate was washed with 1.0 mL cold (4°C) acetone twice, each washing was followed by centrifugation and decanting the residual acetone. The washed protein precipitate was placed in a crucible (a sample holder for nitrogen analyzer) and dried under vacuum.

Nitrogen content within the precipitate was determined using a Skalar Primacs SN Total Nitrogen Analyzer (Breda, The Netherlands). The principle behind the nitrogen analysis is based on the Dumas method using EDTA as the standard. Nitrogen content was converted to protein content by multiplying a factor of 6.25. Errors represented are standard deviation of duplicate experiments. The protein concentrations of the respective commercial enzymes analyzed according to this protocol are presented in Table 3.

**Table 3: Concentration of Nitrogenous Compounds in Commercial Enzymes**

| | Total Nitrogen | Protein Equivalent |
|---|---|---|
| | mg/mL | |
| Accellerase 1000 | 8.5±0.1 | 53.1±0.6 |
| Spezyme CP | 13.4±1.1 | 83.5±6.8 |
| Novozyme 188 | 10.6±0.1 | 66.3±0.7 |
| Multifect Xylanase | 5.0±0.3 | 31.0±1.7 |
| Multifect Pectinase | 8.3±0.0 | 51.9±0.2 |

### Free Amino Acids

500 µL of each of the respective solutions were filtered (Millipore Centricon), 20uL of the filtered elute was derivatized with AccQ Tag (Waters), 10% of the total derivatized sample was injected into the HPLC system.

### Protein Amino Acids

The three solutions were dried under vacuum (SpeedVac, Savant) and hydrolyzed with 6N HCl at vapor phase at 100°C for 24 hrs. The hydrolyzed dry samples were solubilized in 100 µL of 20mM HCl and 10µL of the mixture was derivatized with AccQTag (Waters). 10% of the derivatized mixture was injected into a Nova Pak C18 (3.9mm×150mm; Waters).

### Total Nitrogen Content

Nitrogen contents of the dry untreated CS, AFEX-treated CS, solid residue, enzyme solution and AFEX-CS hydrolysate were determined using a Skalar Primacs SN Total Nitrogen Analyzer (Breda, The Netherlands). Liquid samples (1 mL) were dried at 110°C overnight prior to the analysis. The nitrogen analysis is based on the Dumas method using EDTA as the standard. Nitrogen content of the samples was calculated by dividing nitrogen content (g) of the analyzed materials by weight or volume of the samples.

### Minerals

Trace elements were measured by inductively-coupled-plasma mass spectrometry (ICP-MS) in the MSU Department of Geological Sciences.

### Liquid Samples

Approximately 1 mL of liquid sample was digested on a hot plate, sub-boiling, in acid-cleaned Teflon savillex beakers using 1.9 mL Optima nitric acid and 0.1 mL trace metal clean hydrofluoric acid for 24 hours. After digestion 0.250 mL of trace metal clean 30% hydrogen peroxide was added and the sample evaporated to near dryness on a hotplate. Samples were then brought up to final volume with 5 mL of 2% Optima nitric acid, visual inspection showed a complete digestion of all samples. This solution was run in the ICP-MS for full mass scan analyses.

### Solid Samples

Approximately 100 mg of solid samples was added to 5 mL of Optima nitric acid in an acid cleaned Teflon Savillex vial and sonicated for 60 minutes to homogenize the sample. Then the samples were digested, sub-boiling, overnight on a hot-plate. After approximately 24 h, 0.1 mL of trace metal clean hydrofluoric acid and 1 mL of trace metal clean 30% hydrogen peroxide was added and digested for another 24 hours. Finally the samples were allowed to evaporate to near dryness and taken up to a final volume of 5 mL with 2% Optima nitric acid. This solution was run in the ICP-MS for full mass scan analyses.

### Major Element Analysis

The major elements analyzed include potassium (K), magnesium (Mg), calcium (Ca), phosphorus (P), and sodium (Na) samples were diluted 1:300 prior to analysis. For trace element analysis: chromium (Cr), cobalt (Co), nickel (Ni), copper (Cu), arsenic (As), cadmium (Cd), lead (Pb), molybdenum (Mo), uranium (U), manganese (Mn), zinc (Zn), selenium (Se), barium (Ba) and iron (Fe) samples were run without dilution.

### Vitamins

Five vitamins useful for industrial fermentations were analyzed using a LC/MS/MS (Quattro Micro, Waters) using a Waters Symmetry C-18 column. The mobile phase was run at 0.3 mL/min with a gradient of 1 mM perfluoroheptanoic acid and acetonitrile. Mass spectra were acquired for 6 min using electrospray ionization in positive ion mode. The capillary voltage, extractor voltage and RF lens voltage was set at 3.17 kV, 4.00 V and 0.3 V, respectively. The source temperature and desolvation temperature were at 110°C and 350°C. The desolvation gas flow was set at 400L/hr. Collision energies and source cone potentials were optimized for each transition using Waters QuanOptimize software. Data was acquired with MassLynx 4.0 and processed with QuanLynx software.

### Fermentation on water extracts from CS and RS

Water extracts of AFEX-corn stover and AFEX-rice straw at 9% Solids Loading Equivalent (SLE) were prepared according to the method described in Lau, M. W. et al., The impacts of pretreatment on the fermentability of pretreated lignocellulosic biomass: a comparative evaluation between ammonia fiber expansion and dilute acid pretreatment, Biotechnol Biofuels, vol. 2, 30 pp, 2009 (Epub Date: December 8, 2009).

Glucose and phosphate buffer (salts) were added into the water extract to a final concentration of 100 g/L and 0.1 M, respectively. The pH of the water extract was adjusted to 5.5. Seed culture of S. *cerevisiae* 424A(LNH-ST) was prepared by inoculating frozen stock to Yeast Extract Phosphate (YEP) media (5 g/L yeast extract + 10 g/L tryptone + 30 g/L glucose + 20 g/L xylose) and the culture was grown microaerobically at 30C for about 18hr. The grown culture was used to inoculate the water extracts at an initial cell density of 2.0 units at OD600nm. Control experiments in Yeast Extract Phosphate (YEP) and Yeast Nitrogen Base (YNB) were conducted in parallel. These fermentations were conducted in triplicate at 10 mL working volume in 15 mL screw capped vials. Samples were taken at designated periods.

### Two-stage Ethanol Fermentation

Frozen glycerol stocks of native S. *cerevisiae* (ATCC 4124) were inoculated into 2% w/w corn steep liquor (CSL) media, supplemented with 20 g/L glucose and grown for 18hr. The grown seed culture was used as inoculum for a first-stage fermentation on the 6% glucan loading AFEX-CS enzymatic hydrolysate at a working volume of 80 mL with an initial cell density equivalent to 0.05 unit OD600nm. The fermentation was conducted for 15 hr and the broth was transferred to 50 mL conical tubes (Falcon, BD) to allow cell separation by sedimentation at 30°C for 3 hr. The clarified liquid hydrolysate was pipetted from the tubes to the unbaffled Erlenmeyer flask.

Seed cultures of the xylose-fermenting *S. cerevisiae* 424A(LNH-ST) were prepared by inoculating the frozen stock into 3:10 diluted 6% glucan loading enzymatic hydrolysate supplemented with 2% weight/weight (w/w) of CSL and the culture was grown for 18hr. The cells were harvested and inoculated into the enzymatic hydrolysate from a first-stage fermentation at a working volume of 70mL with an initial cell density of 25 unit OD600nm. The fermentation was conducted for another 48hr. The S. *cerevisiae* in the seed cultures and the two-stage ethanol fermentation were grown microaerobically in unbaffled Erlenmeyer flasks at 30°C, 150 rpm, pH 5.5 according to the method described in Lau, M. W. et al, Cellulosic ethanol production from AFEX-treated corn stover using Saccharomyces cerevisiae 424A(LNH-ST), Proc Nat'l Acad Sci USA, Vol. 106, Issue 5, pp. 1368-73 (2009) (Epub Date: December 8, 2009). Samples were taken at designated times and cell density was measured as described in Lau, M. W. et al., Ethanolic fermentation of hydrolysates from ammonia fiber expansion (AFEX) treated corn stover and distillers grain without detoxification and external nutrient supplementation, Biotechnology and Bioengineering, vol. 99, Issue 3, pp. 529-539 (2008) (Epub Date: January 24, 2009).

### Trichoderma reseei RUT-C30 Fermentation

Frozen glycerol spore stocks of RUT-C30 were inoculated into 2%w/w CSL supplemented with 20 g/L glucose at pH 5.5 (50 mM phosphate buffer). The preculture (50mL) was grown in a 250 mL baffled flask at 30°C, 200rpm for 48hr. During the enzyme induction phase, 18% solids-loading-equivalent (SLE) AFEX-CS water extract (loaded at 27.8% v/v) and/or finely-ground (passed through 0.5mm screen) AFEX-CS (1% w/v) were added into the preculture (60% v/v) as the enzyme inducer. Distilled water and phosphate buffer were added to the respective mixtures to achieve a final volume of 50 mL. Additional CSL equivalent to a final concentration of 1% w/w CSL was supplemented after 24hr of induction. The fermentation broth was conducted at 30°C ,120 h and pH was adjusted to 5.5 every 24hr by adding HCl. The working volume of the fermentation during induction phase was 50 mL. Upon completion of the induction phase, the fermentation broth was centrifuged at 2,500 x g for 30 min. The cell-free fermentation broth was used to conduct enzymatic hydrolysis at 1% glucan loading AFEX-CS.

The cellulase and/or hemicellulase enzymes were separated from the other background protein at smaller molecular weight by using a FPLC system (GE Healthcare, Buckinghamshire, United Kingdom) equipped with a 51 ml HisPrep 26/10 desalting column (GE Healthcare, Lot #17-5087-01). The concentration of fractions that contained the cellulase and/or hemicellulase enzymes was determined by BCA assay (Pierce Biotechnology, Rockfort, IL). The original concentration was calculated by taking into the account of the dilution factors involved.

To compare the induction efficiency of AFEX-CS liquid extract relative to lactose, RUT-C30 precultures (60% v/v) were treated either with 18% solids-loading-equivalent (SLE) AFEX-CS liquid extract (loaded at 27.8% v/v) or with 4.17 g/L of lactose. Other fermentation procedures and parameters were kept identical to facilitate comparison. The initial carbohydrate concentration of both inducers was the same (4.17 g/L).

### Enzymatic Hydrolysis of AFEX-CS at 1% Glucan Loading

RUT-C30 fermentation broth was diluted by a factor of 1:6 (1 part of broth + 5 part of distilled water). The diluted broth was used to conduct enzymatic hydrolysis on AFEX-CS at 1.0% glucan loading for 24hr at pH 4.8, 50 °C. The AFEX-CS was finely ground and passed through a 0.25mm screen (Ultra Centrifugal Mill ZM 200, Retsch, Germany). Accellerase at varying dosages (0.0, 1.0, 2.0 mg protein/g dry AFEX-CS) was added to the diluted broth to investigate the need for exogenous enzyme supplementation. Control experiments using commercial enzymes mixture were conducted for comparison. The enzyme mixture consisted of Accellerase 1000 (120 mL/kg CS), Multifect Xylanase (6.2 mL/kg CS) and Multifect Pectinase (4.3 mL/kg CS).

### Analytical methods

The levels of oligosaccharides were quantified using NREL-LAP-014, a method based on acid hydrolysis. Glucose, xylose, arabinose and ethanol are quantified using a HPLC system equipped with Biorad Aminex HPX-87H column as described in Lau et al., Biotechnology and Bioengineering 99(3): 529-39 (2008).

### Enzymatic hydrolysis of AFEX-CS at 6% Glucan Loading

The enzymatic hydrolysis (2.0 kg total saccharification) was conducted in a 3L bioreactor (Applikon, Biobundle, Foster City CA) at 50°C, pH 4.8 for 96 hr. To achieve proper liquefaction and stirring throughout the enzymatic hydrolysis, both AFEX-CS and commercial enzymes were fed batch wise. AFEX-CS was fed in 5 batches (6.0%, 3.0%, 3.0%, 3.0%, 3.0% solids loading) with intervals of 2, 1, 1, 1.5, 1.5 hr between the respective additions. Regarding enzyme feeding, two-thirds of the total enzymes was added during the first 8 hr of the feeding (1/3, at 0 hr; 1/6, at 4hr; 1/6, at 8hr). The remaining one-third of the enzymes was fed into the reactor over the subsequent 40 hours (1/6, 8-24 hr; 1/6, 24-48hr), the feeding was distributed evenly every 60min. A total protein loading of 7.4 mg protein/g biomass was used. The commercial enzymes and their respective dosages used were Accellerase 1000 (120 mL/kg CS), Multifect Xylanase (6.2 mL/kg CS) and Multifect Pectinase (6.2 mL/kg CS). The protein concentration of the commercial enzymes was analyzed and the mass balance for enzymatic hydrolysis was constructed as described in Lau, M. W. et al, Cellulosic ethanol production from AFEX-treated corn stover using Saccharomyces cerevisiae 424A(LNH-ST), Proc Nat'l Acad Sci USA, Vol. 106, Issue 5, pp. 1368-73 (2009) (Epub Date: December 8, 2009).

### Trichoderma Extracellular Protein Isolation for Proteomics

The extracellular proteins from the fungal broths were isolated using chloroform/methanol precipitation method as described by Wessel & Flügge, Analytical Biochemistry 138(1): 141-143 (1984) and Jiang et al., Journal of Chromatography A 1023(2): 317-320 (2004). Four parts volume cold methanol was added to one part volume of the broth and vortexed well. One part volume cold chloroform followed by three part volumes cold water was then added to the mixture and vortexed again. The mixture was centrifuged at 15,000 g at 4 °C for 10 min following which the aqueous (top) layer was discarded and four part volume cold methanol was added. The mixture was centrifuged at 15,000 g at 4 °C for 30 min following which the liquid supernatant was carefully removed without disturbing the precipitated protein pellet. The protein pellet was air-dried overnight and re-dissolved in SDS-PAGE sample buffer (NuPAGE^{®} LDS Sample Preparation Buffer, Invitrogen, CA) prior to proteomics analysis.

### Proteomics and Protein Homolog Identification

Re-dissolved proteins (∼400µg) were loaded on to a SDS-PAGE gel (NuPAGE^{®}, Invitrogen, CA) and electrophoresis was carried out at 50 V for 15 min to stack up the proteins within the gel. Gel bands were then cut out and subjected to in-gel tryptic digestion (Shevchenk et al., Anal. Chem. 68(5): 850-58 (1996)). The extracted peptides were resuspended into a solution of 2% Acetonitrile and 0.1% Trifluoroacetic Acid to 20 µl volume. From this solution, 10 µl were automatically injected by a Waters nanoAcquity Sample Manager (www.waters.com) and loaded for 5 minutes onto a Waters Symmetry C18 peptide trap (5µm, 180µm x 20mm) at 4µL/min in 2% Acetonitrile/0.1% Formic Acid. The bound peptides were then eluted using a Waters nanoAcquity UPLC (Buffer A = 99.9% Water/0.1% Formic Acid, Buffer B = 99.9% Acetonitrile/0.1 % Formic Acid) onto a Michrom MAGIC C18AQ column (3u, 200 Angstrom, 100µm x 150mm, www.michrom.com) and eluted over 60 minutes with a gradient of 2% B to 30% B in 46min, spiked to 90%B at 47 minutes and equilibrated back to 5% B after 49 minutes at a flow rate of 1 µl/min. Eluted peptides were sprayed into a ThermoFisher LTQ-FT Ultra mass spectrometer (www.thermo.com) using a Michrom ADVANCE nanospray source. Survey scans were taken in the FT (25000 resolution determined at m/z 400) and the top ten ions in each survey scan were then subjected to automatic low energy collision induced dissociation (CID) in the LTQ. The resulting MS/MS spectra are converted to peak lists using BioWorks Browser v3.3.1 (ThermoFisher) using the default LTQ-FT Ultra parameters and searched using the Mascot search algorithm v2.3 (www.matrixscience.com) against fungi protein entries from NCBI, downloaded 11-13-2009, and against the *Trichoderma reesei* protein database, v2.0, downloaded from the DOE Joint Genome Institute (JGI). Mascot parameters for all databases allowed for up to 2 missed tryptic sites, fixed modification of carbamidomethyl cysteine, and variable modification due to oxidation of methionine. The peptide and MS/MS fragment tolerance was ± 10 ppm (monoisotopic) and 0.60 Da (monoisotopic), respectively. The Mascot output was then analyzed using Scaffold (www.proteomesoftware.com) to probabilistically validate protein identifications using the ProteinProphet computer algorithm (Nesvizhskii et al., Anal. Chem. 75(17): 4646-58 (2003)). Minimum criteria for positive protein assignment were at least two peptides and >95% confidence filter as determined by Scaffold. Uncharacterized and/or putative protein sequences were BLAST against the UniprotKB database to identify homology to similar proteins from other microbes.

### Additional results and analysis

### Nutrient Contents and Balances During Processing of AFEX-treated corn stover (AFEX-CS)

Amino acid, trace element and vitamin content of the enzymatic hydrolysate at 6% cellulose loading was analyzed and quantified. The results are shown below in Tables 4 and 5. The enzymatic hydrolysate contained 800±50 mg/L ammonia and 1231±44 mg total amino acids, of which 16% by weight were in the form of free amino acids as shown in Table 4 below. Glutamic acid (Glu), glycine (Gly) and alanine (Ala) were the three most abundant amino acids found in the hydrolysate. The amino acid concentration is higher than that of a typical malt wort for brewery applications (800-900 mg/L total amino acids).

**Table 4: Amino Acid Concentration of AFEX Corn Stover (AFEX-CS) Enzymatic Hydrolysate**

| Components | AFEX-Hydrolysate (mg/L) | |
|---|---|---|
| | Free | Total |
| NH⁴⁺ | 750±50 | |
| Asparagine (Asp) | 8.4±1.7 | 75.9±1.7 |
| Glutamic Acid(Glu) | 0.0±2.4 | 133.8±2.4 |
| Serine (Ser) | 16.8±3.8 | 104.2±3.8 |
| Glycine (Gly) | 5.2±5.8 | 127.2±5.8 |
| Histidine (His) | 4.5±2.3 | 34.3±2.3 |
| Threonine (Thr) | 17.6±4.6 | 98.9±4.6 |
| Arginine (Arg) | 17.1±3.2 | 55.0±3.2 |
| Alanine (Ala) | 11.6±2.9 | 110.2±2.9 |
| Proline (Pro) | 30.4±2.3 | 108.7±2.3 |
| Threonine (Thr) | 30.0±2.5 | 28.6±2.5 |
| Valine (Val) | 9.9±2.2 | 68.8±2.2 |
| Methinonine (Met) | 2.6±1.9 | 19.4±1.9 |
| Isoleucine (Ile) | 7.6±2.2 | 55.4±2.2 |
| Leucine (Leu) | 0.0±3.8 | 93.6±3.8 |
| Lysine (Lys) | 18.4±1.3 | 25.7±1.3 |
| Phenylalanine (Phe) | 15.7±3.8 | 91.6±3.8 |
| Total | 195.8±28.3 | 1231±43.8 |

Ten trace elements known to be useful in microbial growth were in excess compared to values suggested in the literature for yeast fermentation. See Walker, G.M. in Advances in Applied Microbiology, Vol. 54. (eds. A.I. Laskin, J.W. Bennet & G.M. Gadd) (Elsevier Academic Press, New York, NY; 2004) (hereinafter "Walker"). Magnesium (269±6mg/L) was considered to be at a sufficient level as shown in Table 5. Concentrations of panthothenic acid, pyridoxine, nicotinic acid, and biotin exceeded levels seen in a typical malt wort. The level of thiamine (0.4 µM) is slightly lower than the "reference" concentration range of 0.57-2.83 µM. See Walker.

AFEX-CS was the predominant source of protein, trace elements and vitamins in the enzymatic hydrolysate. The contribution of commercial enzymes to the nutrient levels was very low.

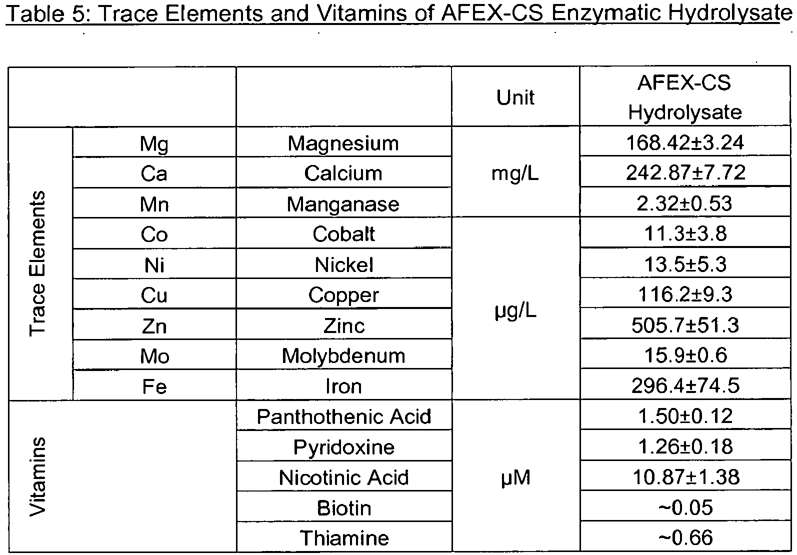

Table 6 shows the full mineral analysis results for untreated CS, AFEX-CS and residual solids after enzymatic hydrolysis.

**Table 6: Full mineral analysis results for untreated CS, AFEX-CS and residual solids after enzymatic hydrolysis**

| Concentration (mg/kg) | Untreated Kramer Corn Stover | AFEX Kramer Corn Stover | Residual Solid after 6% Glucan Enzymatic Hydrolysis |
|---|---|---|---|
| Cr | 3.4E+01 | 1.2E+01 | 1.2E+00 |
| Co | 5.8E-01 | 1.2E-01 | 7.5E-02 |
| Ni | 1.6E+01 | 5.3E+00 | 1.3E+00 |
| Cu | 3.9E+00 | 3.4E+00 | 9.5E+00 |
| As | 1.0E-01 | 7.2E-02 | Not Detected |
| Cd | 9.1E-02 | 7.5E-02 | 2.0E-01 |
| Pb | 1.8E+00 | 1.0E+00 | 2.4E+00 |
| Mo | 4.9E+00 | 1.9E+00 | 1.9E+00 |
| U | 7.9E-02 | 6.0E-02 | 1.8E-01 |
| Mn | 1.5E+01 | 1.2E+01 | 8.2E+00 |
| Zn | 8.5E+00 | 8.1E+00 | 1.9E+01 |
| Se | Not Detected | Not Detected | Not Detected |
| Ba | 2.8E+01 | 2.5E+01 | 2.9E+01 |
| Fe | 1.8E+02 | 1.2E+02 | 1.1E+02 |
| Ca | 1.9E+03 | 1.8E+03 | 1.8E+03 |
| P | 6.3E+02 | 6.5E+02 | 8.5E+02 |
| Na | Not Detected | Not Detected | 2.0E+02 |
| K | 1.1E+04 | 1.2E+04 | 2.5E+03 |
| Mg | 8.7E+02 | 8.5E+02 | 2.7E+02 |
| Total | 1.5E+04 | 1.5E+04 | 5.8E+03 |

Table 7 shows full mineral analysis results for 9% SLE water extract of AFEX-CS and AFEX-RS.

**Table 7: Full mineral analysis results for 9% SLE water extract of AFEX-CS and AFEX-RS**

| | 9% SLE Water Extract Rice Straw | 9% SLE Water Extract Corn Stover | Concentration |
|---|---|---|---|
| Cr | 8.08 | Not Detected | |
| Co | 21.92 | 82.73 | |
| Ni | 61.16 | 29.79 | µg/L |
| Cu | 143.47 | 89.41 | |
| As | 64.94 | Not Detected | |
| Cd | Not Detected | Not Detected | |
| Pb | Not Detected | 17.65 | |
| Mo | 33.33 | 23.21 | |
| U | Not Detected | Not Detected | |
| Mn | 12186.09 | 198.19 | |
| Zn | 278.71 | 126.63 | |
| Se | Not Detected | Not Detected | |
| Ba | 334.01 | 465.36 | |
| Fe | 437.18 | 152.98 | |
| P | Not Detected | Not Detected | |
| Na | 37.34 | Not Detected | mg/L |
| Mg | 96.23 | 57.33 | |
| Ca | Not Detected | 73.40 | |
| K | 1.62 | 1.16 | g/L |

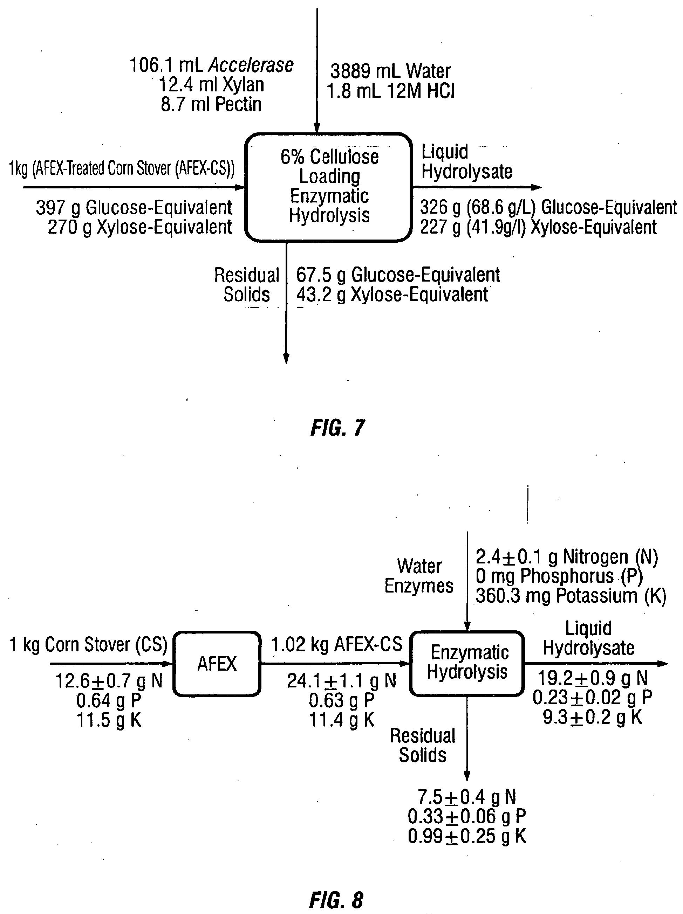

AFEX-CS at 18% solids loading (6% glucan loading) was enzymatically-hydrolyzed using commercially-available enzymes. About 85% of the total carbohydrate was hydrolyzed and solublized in the liquid stream, achieving a total soluble sugars concentration of 110 g/L. See FIG. 7.

The mass balance with respect to total nitrogen (N), phosphorus (P) and potassium (K) confirmed that 80-82% of the total nitrogen and potassium from the AFEX-CS was solubilized into the liquid stream (hydrolysate). However, most of the phosphorus content (64%) was left on the residual solids following enzymatic hydrolysis. See FIG. 8.

### Empirical test on ethanol fermentation using water extract from AFEX-treated CS (AFEX-CS) and AFEX-treated RS (AFEX-RS)

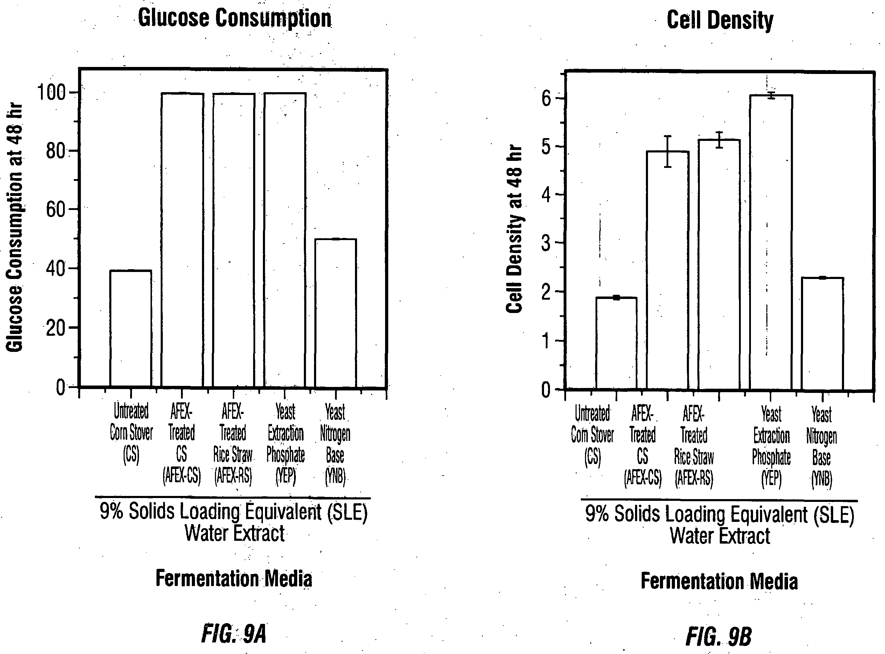

A water extract with a 9% solids loading equivalent, containing partially solubilized biomass constituents, was generated from both AFEX-CS and AFEX-RS. Glucose was added as carbon source to produce a final concentration of 100 g/L. After 48 h of fermentation using S. *cerevisiae* 424A(LNH-ST), glucose in both water extracts was completely consumed. The final yeast cell density (at 48 h) was 4.5-5.5 g dry wt/L. (FIGS. 9A and 9B). These values were greater than fermentation in yeast nitrogen base (YNB; 13.7 g/L), but not as high as in yeast extract + peptone (YEP; 5 g/L yeast extract + 10 g/L peptone). These results indicate that nutrient levels greater than 9% solids loading equivalent from the pretreated CS and RS are sufficiently high to support yeast fermentation. Furthermore, as indicated by the data, nutrients are in excess in a higher solid loading hydrolysate, and thus such a water extract or hydrolysate may support multiple fermentations.

### Two-stage ethanol fermentation using native and recombinant Saccharomyces cerevisiae

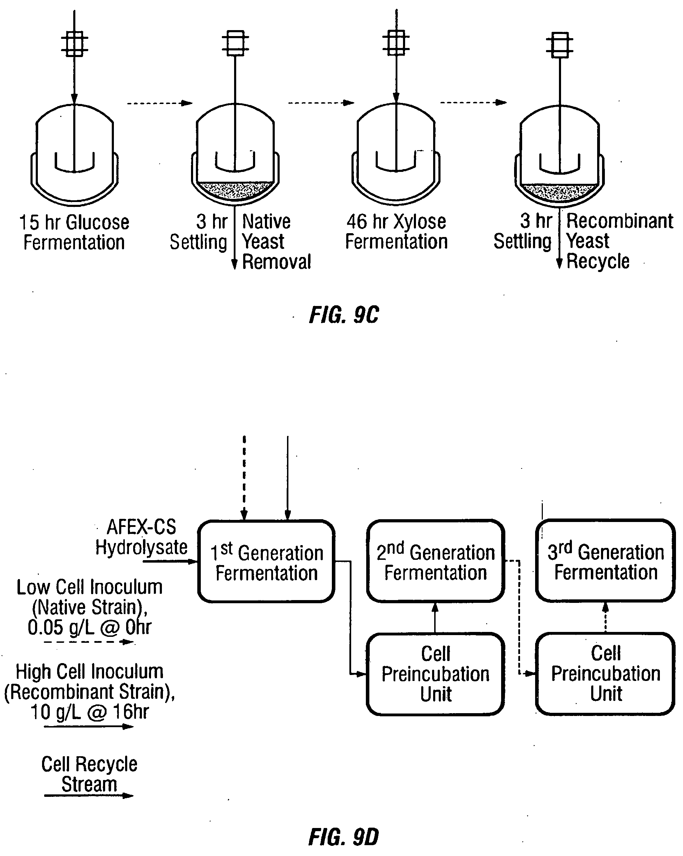

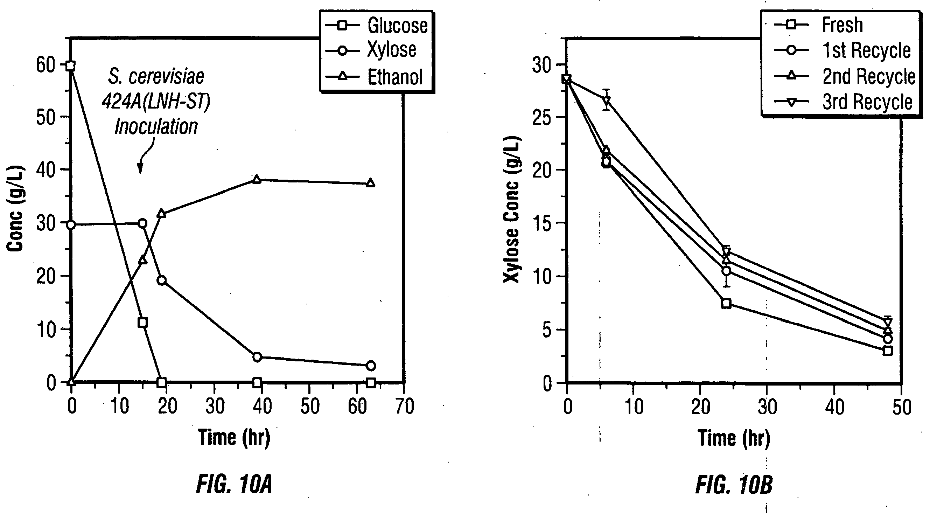

To further investigate the potential of using corn stover at high solids loading to support multiple fermentations, AFEX-CS hydrolysate from 18% w/w loading saccharification was fermented first by native S. *cerevisiae* (ATCC 4124) for 15 hr, before the yeast cells were separated from the hydrolysate by sedimentation for 3 hr (FIG. 9C). In the second stage, high cell density (11.25 g dry wt/L) of recombinant S. *cerevisiae* 424A(LNH-ST) was inoculated to ferment the remaining sugars (FIG. 9C). The glucose fermentation proceeded at a rate of 3.2 g/L/h (0-15hr) and was completed at 18hr. After sedimentation, 4.1 g dry wt/L yeast cells were collected. In the second stage fermentation at high cell density, 87% of the xylose was consumed within the first 24hr of the recombinant S. *cerevisiae* inoculation. A final ethanol concentration of 39 g/L was achieved with metabolic ethanol yield at 92.1% of the theoretical maximum (FIG.10A).

The recombinant S. *cerevisiae* 424A(LNH-ST) cells were recycled and used in three subsequent cycles of fermentation (FIG. 9D). In essence, xylose fermentation using the recycled yeast cells achieved a similar efficiency compared to fresh cells (FIG. 10B). This effectively reduced the need for fresh cells in the successive batches of fermentation without significantly decreasing the xylose fermentation rate, a potentially significant cost savings.

### In-house enzyme production

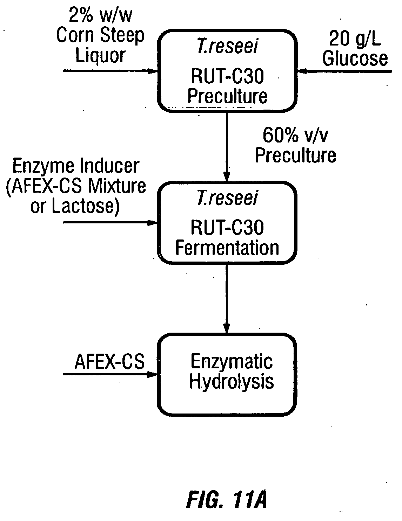

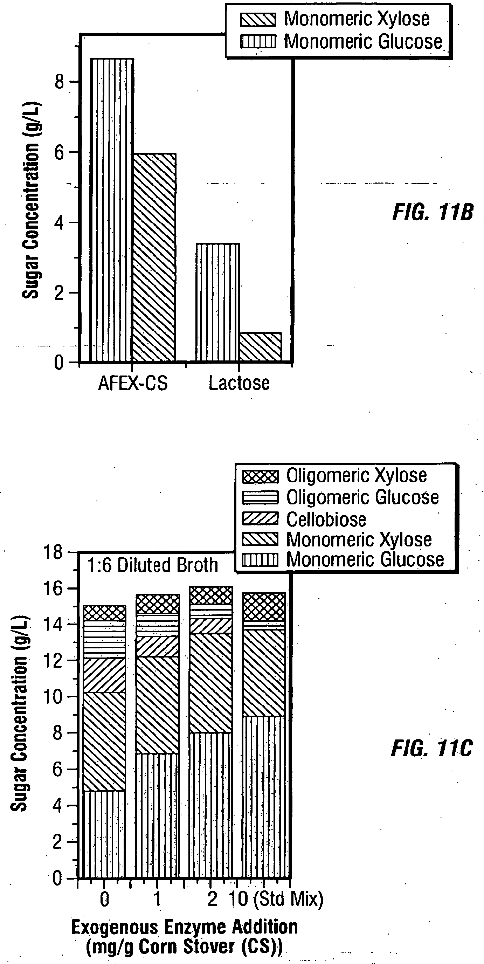

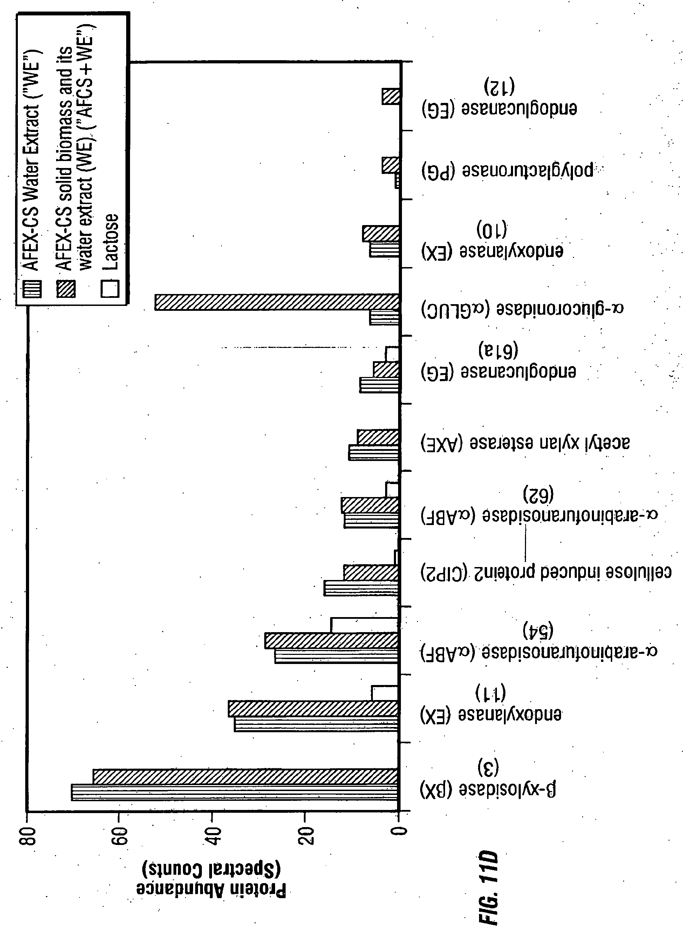

*Trichoderma reesei* RUT-C30 was first cultured in media consisting of 2% w/w corn steep liquor and 20 g/L glucose for 36 hr. The cellulase and/or hemicellulase enzymes were induced for 96 h by a mixture of solids and liquid extract from AFEX-CS (FIG. 11 A). Table 8 shows the enzyme inducers used for *T. reseei* RUT-C30 fermentation for FIGS. 11B, 11C and 11).

**Table 8: Enzyme inducers for T. reseei RUT-C30 fermentation for FIGS. 11B-11**

| FIGURE | AFEX-CS mixture | Lactose (g/L) |
|---|---|---|
| 11B | 5.4% SLE water extract (containing 4.17 g/L equivalent of total sugar) | 4.17 |
| 11C | 5.4% SLE water extract + 1 % w/v AFEX-CS | N/A |
| 11D | (WE): 5.4% SLE water extract (AFCS + WE): 5.4% SLE water extract + 1 % w/v AFEX-CS | 4.17 |

As shown in FIG. 11B, the concentration of cellulase and/or hemicellulase produced was 2.7 g/L which provided 18% w/w enzymatic hydrolysis at 15 mg enzyme protein per gram of corn stover. About 13% of the protein induced by CSL was converted to cellulase and/or hemicellulase enzymes.

The *T. reesei* broth was diluted by a factor of 1:6 and the diluted broth was used to hydrolyze AFEX-CS at 1 % cellulose loading for 24 h with or without additional enzymes (Accellerase 1000). Enzymatic hydrolysis using the 1:6 diluted in-house *T. reesei* broth achieved total soluble sugar yield at 15 g/L within 24hr (85.6% of theoretical maximum yield) (FIG. 11 B). This total sugar yield level is comparable to that of the standard enzyme mixture based on commercial enzymes (15.7 g/L; 89.4%) suggesting that the in-house enzyme production unit can support effective enzymatic hydrolysis at high solids loading (FIG. 11 B). For monomeric sugars, *Accellerase* loading at 1.5-2 mg protein/g corn stover was used to achieve monomeric sugar at similar yields to that provided by the standard mixture, in which 13.7 g/L of total monomeric glucose and xylose was obtained (FIG. 11 B).

It was also found that the AFEX-CS mixture used for the enzyme induction was approximately 2.5-7 times more potent than lactose on a same sugar weight basis. Enzymatic hydrolysis by *T. reesei* achieved 10.4 g/L (59% of total glucose/xylose hydrolysis yield) net increase in sugar yield when AFEX-CS extract was used for induction instead of lactose on a same initial sugar-equivalent basis (FIG. 11 B).

There was little difference in the abundance (within two-fold) of most cellulases (e.g., Cel7A, Cel6A, Cel74A, Cel7B, Cel5A, and β-mannase) expressed by *T. reesei* using AFEX-CS or lactose. However, AFEX-CS treatment of *T. reesei* resulted in a significant increase (> 2-fold in protein abundance) in expression of several endocellulases (Cel61A, Cel12A) and hemicellulases (e.g., β-xylosidase, endoxylanases, α-arabinofuranosidases, CIP2, acetyl xylan esterases, α-glucuronidase, polygalaturonase) that were previously not observed for lactose-induced *T. reesei.* FIG. 11D shows the relative abundance of proteins expressed using AFEX-CS or lactose, where a greater than two-fold difference in abundance was observed relative to the lactose control.

Using AFEX treated corn stover (with AFCS or AFCS+WE as inducer) resulted in significant increase in expression of several new families of endocellulases (Cel61A, Cel12A) and hemicellulases (β-xylosidase, endoxylanases, α-arabinofuranosidases, CIP2, acetyl xylan esterases, α-glucuronidase, polygalaturonase) that are typically missing from the proteome of lactose-induced *Trichoderma reesei* RUT-C30.

Interestingly, using AFEX treated solid biomass along with AFCS water extract was responsible for inducing several enzymes (Cel12A, Cel61A, α-glucuronidase, polygalacturonase, β-galactosidase, α-galactosidase, acetyl esterase and others) that were either not expressed or were present at very low abundances when using AFCS water extract alone. This suggests that the presence of solid pretreated lignocellulosic biomass is useful in inducing expression of a more comprehensive suite of CAZymes than using only soluble inducers isolated from pretreated lignocellulosic biomass.

Expression of α-glucuronidase, α-galactosidase and acetyl esterase only in the presence of solid biomass also suggests there are certain insoluble hemicellulosic components (e.g. branched xylan decorated with glucuronic acid side-chains) trapped in the cell wall matrix that are necessary for inducing these enzymes. Under the AFEX conditions employed, there should be at least 10-15% of intact acetyl esters in corn stover cell walls that are likely responsible for induction and expression of acetyl esterases (JGI#121418). However, another acetyl xylan esterase (JGI#54219) was expressed in similar abundance levels for both AFCS and AFCS-WE but was absent when induced by lactose alone. A 10-15 fold higher levels of CIP2 abundance for AFCS/AFCS+WE compared to lactose alone was also seen.

A significant amount of arabino-xylan oligomers are released from corn stover cell walls after AFEX, which may explain the large increase observed in β-xylosidase expression that is likely induced by these hemicellulose oligomers.

### EXAMPLE 5

### Fed-batch Fermentation of AFEX-CS Enzymatic Hydrolysate using enzyme secreting ethanologen Thermoanaerobacterium saccharolyticum

Fed-batch fermentation was conducted in a custom-made fermenter (NDS Technologies, NJ) equipped with a pH probe. The fermenter temperature was controlled by an external water bath recirculation system. Feeding and pH were controlled by Sartorius A plus system (Goettingen, Germany). Initial volume of the reactor was 120 mL which consisted of 20 mL enzymatic hydrolysate at 18% solids loading, nutrient supplement and distilled water (for dilution). For nutrient supplementation, 1 g yeast extract, 0.5 g peptone, and 10 mL of concentrated stock for solution B, C, D and E (See Table 9) was added.

**Table 9: MTC Media for T. saccharolyticum ALK2 Growth**

| | | Final Conc. |
|---|---|---|
| Solution A | Yeast extract | 10 g/L |
| | Tryptone | 5 g/L |
| | MES (buffer) | 10 g/L |
| Solution B | Citric acid potassium salt | 2.00 g/L |
| | Citric acid monohydrate | 1.25 g/L |
| | Sodium sulfate (Na₂SO₄) | 1.00 g/L |
| | Potassium dihydrogen phosphate (KH₂PO₄) | 1.00 g/L |
| | Sodium bicarbonate (NaHCO₃) | 2.50 g/L |
| Solution C | Urea | 5.00 g/L |
| | Ammonium Chloride (NH₄Cl) | 1.50 g/L |
| Solution D | Magnesium chloride hexahydrate (MgCl₂.6H₂O) | 1.00 g/L |
| | Calcium chloride dehydrate (CaCl₂.2H₂O) | 0.20 g/L |
| | Iron (II) chloride tetrahydrate (FeCl₂.4H₂O) | 0.10 g/L |
| | L-cysteine hydrochloride monohydrate | 1.00 g/L |
| Solution E | Pyridoxamine dihydrochloride | 0.020 g/L |
| | P-Aminobenzoic acid | 0.004 g/L |
| | D-Biotin | 0.002 g/L |
| | Vitamin B₁₂ | 0.002 g/L |

The fermentation media was pH-adjusted to 6.2 with KOH and sparged with nitrogen for about 10 min to create anaerobic condition. The seed culture (10mL) was inoculated to initiate fermentation. Undiluted 18% solids loading enzymatic hydrolysate at pH 6.2 (supplemented with 10 g/L yeast extract and 5 g/L peptone), was used as the feed. Feeding started 4 hr after inoculation at the rate of 4 mL/hr until 180 mL of feed volume was added into the fermenter. Samples were taken at the designated periods. Glucose, xylose, arabinose (in monomeric form) and ethanol were analyzed using HPLC. Oligomeric sugars were analyzed through acid hydrolysis based on NREL Protocol LAP-014.

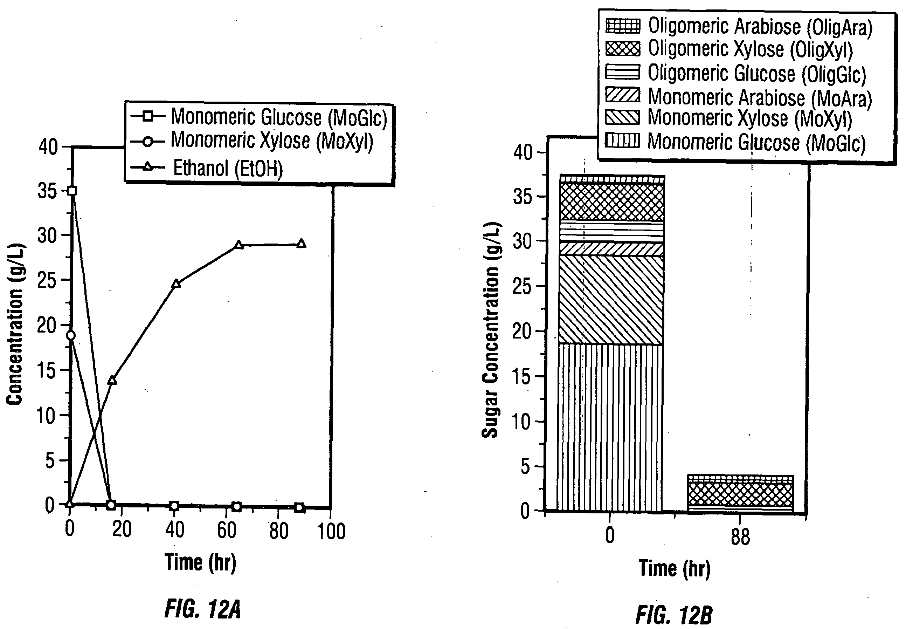

In rich nutrient-supplemented fermentation, nearly 90% of the total sugars (monomers and oligomers) in the hydrolysate were consumed, and a metabolic yield of 0.45 g EtOH/ g consumed sugars was achieved (See FIGS 12A and 12B). Fermentation was completed within 64 hr after inoculation; 15 hr after feeding was concluded. Over 60% of the total oligomeric sugars were consumed in this time period. We demonstrated that ALK2 is able to grow and produce ethanol to 30 g/L at 0.45 g/L/hr (0-64hr) from the hydrolysate containing degradation compounds equivalent to 11.7% solids loading of AFEX-CS.

### EXAMPLE 6

To determine the feasibility of these changes in a commercial refinery, a techno-economic model of the proposed scheme was created and compared to a conventional ethanol production scheme. This model was based on the model developed by NREL. See, for example, Ordonez, C. et al. Bioresour. Technol. 78: 187-190 (2001) and U.S. Patent No. 4,624,805 to Lawhon, issued Nov 25, 1986. The model was adapted to AFEX pretreatment. See, for example, El-Adaway, T. et al. Food Chem. 74: 455-462 (2001).

Changes in the model were made in the section devoted to biological conversion. For example, it was assumed that all upstream and downstream processes would be the same. Hydrolysis yields, enzyme, ethanol and yeast production, and consumption of nutrients were all estimated from experimental data. The ethanol yield used in the analysis was 276 L/ton. This projection was based on the result from the fedbatch fermentation by *Thermoanaerobacterium saccharolyticum* ALK2 on AFEX-CS hydrolysate (Example 4) where about 60% of the cello-oligosaccharides were consumed during fermentation, which increased the overall yield from 246 L/ton to 276 L/ton.

For the changes in the process design, assumptions on power use and equipment and size were taken from the NREL models wherever possible and estimated from literature values when appropriate.

In the initial model, total hydrolysis and fermentation time was set at 168 hours, although current data suggests 72 hours for hydrolysis and 72 hours for fermentation would be sufficient. While simultaneous saccharification and fermentation can occur, it was not explicitly modeled as such. Instead, hydrolysis and ethanol yields were estimated based on earlier experimental data. With improved enzyme formulation, however, yields are expected to be improved over those presented in the literature, and monomeric sugars can increase relative to oligomeric sugars.

All heat and power requirements are assumed as being supplied by burning lignin. No steam was considered for use in the biological conversion step, and all temperature changes were considered to be "mild" changes. Thus, no changes in heat requirements were made relative to the NREL model, as it was assumed that heat integration is possible to supply all changes in energy. For electricity, the added requirements of presses and agitation for the T. *reesei* fermentation were included.

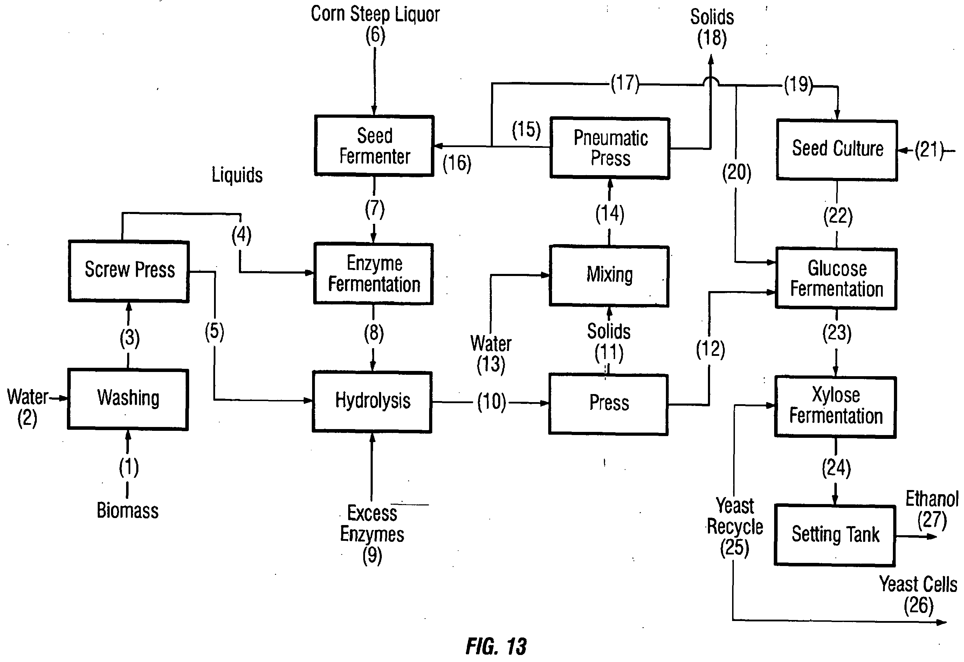

FIG. 13 shows one embodiment of a process flow diagram of the proposed biological conversion computer model, i.e., a simulated operation which has not yet been reduced to practice. In this model, a wash table is used to wet the biomass after AFEX pretreatment, using diluted recycled hydrolysate as the water media. The biomass is dewatered using a screw press. The water effluent is assumed to be rich in oligomeric sugars produced during AFEX (and recycled from hydrolysis), and is thus used to induce the *T. reesei* enzyme production. As an initial approximation, the fungus is assumed to consume 3 g sugar for every gram of enzyme produced. Enzyme production is modeled as a first order reaction of oligomeric sugar with a rate constant of 0.05 h⁻¹. This is sufficient to produce 76% of the enzymes required for lignocellulosic hydrolysis. Total *T. reesei fermentation* time is assumed initially to be 96 hours.

In this model a total of 10 g corn steep liquor (CSL) is consumed per kg biomass to provide the nutrients necessary for *T. reesei* growth and enzyme production. Cellulase and/or hemicellulase enzyme loading of 3-6 mg/g is assumed. Likewise, the biomass contains approximately 6 g nitrogen per kg biomass in the form of acetamide, nearly four times as much nitrogen as required for enzymes. Acetamide is not consumed by *T. reesei* but is by other organisms. Thus, if the fungus can be modified to consume acetamide and can be adapted more fully to AFEX-treated corn stover, then much lower nutrient supplementation is required.

After enzymatic hydrolysis, a pneumapress is used to separate the solids and liquids. This press uses compressed air to force more water out of the biomass, reducing the moisture content to 50% of the total weight. This package is used in the NREL model after distillation, and the same economic assumptions are used here. Because no additional solubilization of biomass occurs after hydrolysis, the cost of the pneumapress is no different in this model than in the NREL model. The liquid released from the press is used as the fermentation media. However, the insoluble biomass still retains some water, which includes hydrolyzed sugars. To ensure that all hydrolyzed sugars are used, the biomass is rinsed with fresh water and then dewatered using a filter press. This residue then exits the process and is burnt for heat and power. The rinsed water is separated into multiple streams. Much of the water is used as the *T. reesei* fermentation media and as the rinse water for obtaining *T. reesei* induction. The remaining water is used as a seed culture for yeast fermentation or combined into the fermentation media.

Fermentation is separated between glucose and xylose fermentation. A settling tank is placed in between glucose and xylose fermentation to recover the yeast. As a first approximation, a residence time of 3 hours is used to settle 95% of the yeast, based on experimental data. The settled yeast is dried in a tunnel dryer before being sold. This energy was assumed to be in the form of steam, and would reduce electricity production by 30% of the total energy requirement. Glucose fermentation time was 15 hours and xylose was 46 hours. After xylose fermentation, another settling tank is used to recycle the yeast, while the fermentation broth is then sent to distillation.

The feedstock is corn stover, and the composition is based on equivalent monomeric sugar content. After AFEX pretreatment, some of the carbohydrates are converted to oligomeric sugars, which are used to induce enzyme production. During cellulose hydrolysis, 18% solid loading is assumed, as it is sufficient to produce 40 g/L ethanol (7). For simplicity, it is assumed that both in-house enzymes and exogenous enzymes have the same activity on all carbohydrates, and thus a constant 10 g/kg enzymes is added regardless of the source. In reality, a constant activity would be added, which may mean different amounts of enzymes, depending on the scale of in-house production. During hydrolysis, it is expected that most of the enzymes are deactivated by permanently binding to the biomass. In this study, 90% of the enzymes were assumed to be deactivated, and thus any recycled enzymes represent only a small fraction of the total.

For fermentation, it is assumed that monomeric glucose is completely consumed, as demonstrated in experimental data. No oligomeric sugars are consumed, and maximum xylose consumption is only 80% of the total sugar present. In addition, total xylose consumed is based on a linear rate of 0.05 g sugar per g yeast per hour. The experiments presented here suggest that xylose consumption is nearly linear at high cell density, and approximately 80% of the sugar is consumed. When no cell recycle is performed, yeast growth is only present during glucose fermentation. Some cell growth is present during xylose fermentation at high cell density, but it is minor. Glucose fermentation is assumed to be slightly more efficient at producing ethanol, with a metabolic ethanol yield of 0.48 g ethanol per g glucose compared to 0.45 g ethanol per grams of xylose consumed. Arabinose hydrolysis and fermentation is assumed to be identical to xylose, and thus all model data is in total pentoses.

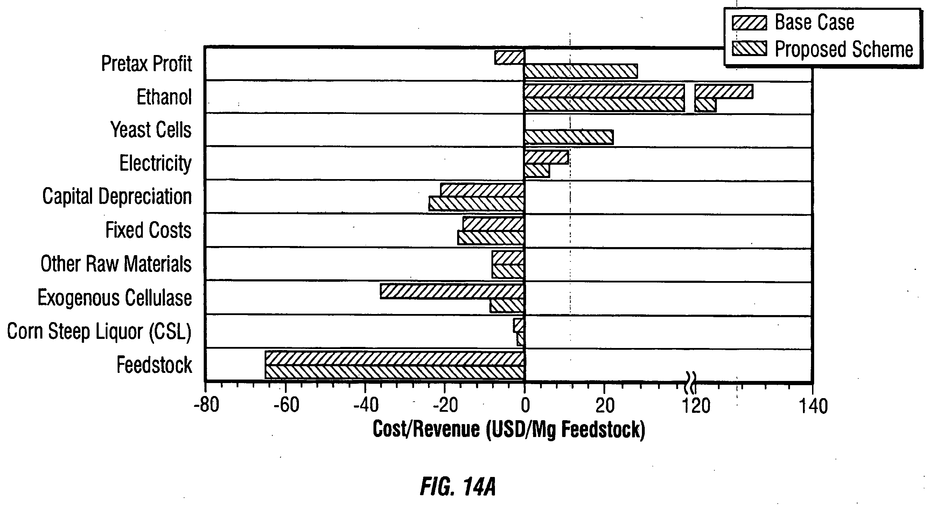

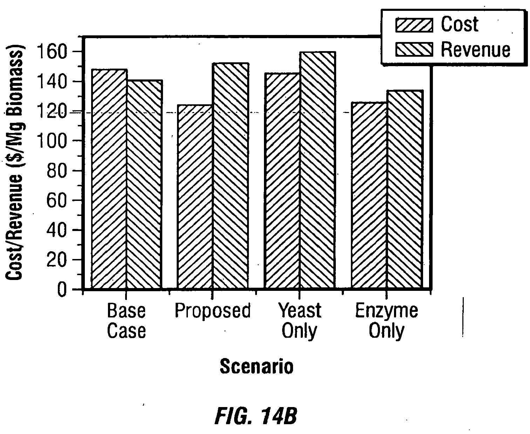

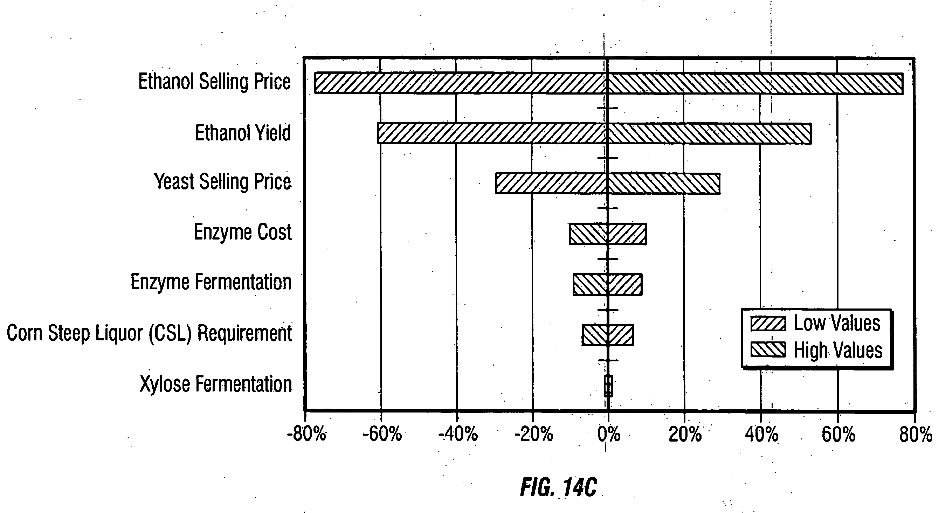

FIG. 14A-14C show the effect of changing variables on the profitability of the proposed approach. Both yield and selling price of ethanol dominate the economics of this approach, as ethanol accounts for over 80% of the revenue in the proposed approach. Likewise, the selling price of native yeast can have an impact, as the overall margin in the base case ($28/mg) is similar to the total revenue of yeast ($22/mg feedstock). Interestingly, the buying price of enzymes does not greatly impact the profit, as a 33% increase in the price translates to a 10% decrease in profit. Thus, despite being a major unknown factor in cellulosic ethanol production, by producing the majority of enzymes on site the influence of enzyme costs decreases. Other considerations, including xylose fermentation time, *T. reesei* fermentation residence time, and nutrient requirements for *T*. reesei fermentation, did not have large impacts on the profitability of the refinery, suggesting that the general approach is feasible and robust. All major process assumptions are shown in Table 10 below.

**Table 10: Process Assumptions**

| | Low | Standard | High | Unit |
|---|---|---|---|---|
| Xylose fermentation time | 12 | 24 | 36 | hours |
| CSL Requirement | 0 | 10 | 20 | g/kg BM |
| *T. reesei* fermentation time | 60 | 96 | 132 | hours |
| Enzyme cost | 2400 | 3600 | 4800 | $/mg enzyme |
| Yeast selling price | 500 | 800 | 1100 | $/mg yeast |
| Ethanol yield | 236 | 275 | 311 | L/mg BM |
| Ethanol selling price | 1.4 | 1.7 | 2 | $/gal EtOH |

### EXAMPLE 8 (PROPHETIC)

Additional testing with other biomass materials will be performed. It is expected that the aqueous extract will stimulate the growth and performance of potential consolidated bioprocessing organisms such as *Clostridium thermocellum.*

### Conclusion

The various embodiments described herein provide for developing microbial growth stimulants from mildly alkaline aqueous extracts from AFEX treated biomass. In one embodiment, a method comprising extracting solubles from pretreated lignocellulosic biomass (e.g., corn stover) with a cellulase enzyme-producing growth medium *(T. reesei),* in the presence of using water and an aqueous extract as a growth medium for the cellulase enzyme-producing fungus *(T. reesei),* is provided. In one embodiment, the pretreated lignocellulosic biomass is ammonia fiber expansion pretreated lignocellulosic biomass.

The testing described in Examples 1-3 was directed towards developing microbial growth stimulants (MGS) from mildly alkaline aqueous extracts from pretreated biomass, such as AFEX treated biomass, such as with a cellulase enzyme. Conventional methods contemplate washing, detoxifying and/or supplementing the AFEX treated biomass with nutrients in order to provide suitable end products useful as starting materials at a biofuel production facility. In contrast, the embodiments herein utilize nutrients contained within the biomass itself, such as protein and minerals during enzyme production and fermentation. As such, the various embodiments described herein provide a new paradigm in the biomass industry in which AFEX-treated biomass can not only serve as the source of carbon and nitrogen in a biofuel production facility, but can also provide a source of other nutrients, such as protein and minerals without washing, detoxification or nutrient supplementation.

Simultaneously achieving economic, environmental and social sustainability is a major challenge for the emerging renewable liquid fuel industry. We approach this problem by demonstrating a cellulosic biorefinery paradigm which produces ethanol and food precursor using lignocellulosic biomass as the exclusive source for carbohydrate and minerals. Enzymatic hydrolysate from Ammonia Fiber Expansion (AFEX)-pretreated corn stover at 18%w/w solids loading was found to be nutrient-rich. This hydrolysate was fermented completely within 48 hr in two stages to produce ethanol and native yeast cells as a coproduct. In-house cellulase and/or hemicellulase enzyme production unit using AFEX-pretreated corn stover as an inducer eliminates the requirement for exogenous enzyme. The inducer mixture was 2.5-7 times more potent than lactose, a common commercial enzyme inducer, on same sugar-weight basis. Economic analysis based on the proposed paradigm indicated substantial improvements in profit margins relative to the 2005 NREL economic model which largely attributed to the value of native yeast cells and the reduction of cellulase cost through the in-house production.

## Claims

1. A method comprising:
treating plant biomass with an ammonia pretreatment to provide an ammonia-pretreated plant biomass, wherein the plant biomass is from one or more plants, and wherein the biomass contains solubles, or solubles and solids;
extracting at least a portion of the solubles from the ammonia-pretreated plant biomass with a solvent to produce a soluble extract and an extracted ammonia-treated plant biomass, wherein the solvent is water; and
using the soluble extract as a stimulant to stimulate growth and promote cellulase and/or hemicellulase enzyme synthesis in an enzyme-producing microorganism.

2. The method of claim 1, wherein the soluble extract comprises proteins, vitamins, simple sugars, oligosaccharides, lipids, trace elements or combinations thereof, and/or wherein the enzyme-producing microorganism is selected from yeast, bacteria, fungi, and combinations thereof.

3. The method of claim 2, wherein the ammonia pretreatment comprises ammonia fiber expansion.

4. The method of claim 1, wherein at least one of the one or more plants is a monocot selected from grass, corn stover, sorghum, sugarcane bagasse, wheat, rice, maize, and a combination thereof.

5. The method of claim 4, wherein at least a portion of the extracted ammonia-pretreated plant biomass is provided to a bio-product production unit as a stimulant, wherein the stimulant is a microbial growth stimulant and/or an agent inducing enzyme production from a microorganism, and the method further comprises hydrolyzing the portion of the extracted ammonia-pretreated plant biomass with an enzyme to generate an enzymatic hydrolysate slurry, wherein the enzyme is an enzyme secreted by the enzyme-producing microorganism.

6. The method of claim 5, further comprising:
mechanically processing the enzymatic hydrolysate slurry to produce a liquid enzymatic hydrolysate comprising a fermentable sugar mixture; and
optionally fermenting the liquid enzymatic hydrolysate to generate a bio-product.

7. The method of claim 1, wherein the solubles are extracted under alkaline conditions having a pH between 7 and 12.

8. The method of claim 1, wherein the method is performed in a bio-product production facility, the bio-product production facility including a bio-product production unit and an enzyme production unit in communication with the bio-product production unit, wherein the bio-product production facility utilizes the extracted ammonia-pretreated biomass to produce a bio-product in the bio-product production unit and contains the enzyme-producing microorganism in the enzyme production unit, the enzyme production unit optionally comprising the extracted ammonia-pretreated biomass, a nutrient-containing byproduct and/or the soluble extract of the bio-product production unit.

9. The method of claim 8, wherein the bio-product is a biofuel or biochemical.

10. The method of claim 9, wherein the biofuel is ethanol and the ammonia pretreated biomass is ammonia pretreated corn stover.

11. The method of any one of claims 8 to 10, wherein the enzyme is produced in an enzyme production unit comprising an enzyme-producing microorganism, an ethanol production residue, and the soluble extract.

12. The method of claim 11, wherein the ammonia-pretreated plant biomass is ammonia-pretreated corn stover and the ethanol production residue is corn stover.

13. The method of any one of claims 5 to 9, further comprising combining the ammonia-pretreated plant biomass with the portion of the extracted ammonia-pretreated plant biomass to form a combined stream prior to providing to the bio-product production unit.

14. The method of claim 13, wherein the enzyme production unit further comprises a diluted pretreated biomass hydrolysate.

15. The method of claim 14, wherein the enzyme-producing microorganism is selected from *Trichoderma reesei, Aspergillus awamori, Clostridium thermocellum* and *Thermoanaerobacterium saccharolyticum,* the enzyme is selected from exoglucanases, endoglucanases, β-xylosidases, endoxylanases, α-arabinofuranosidases, cellulose induced protein 2, α-arabinofuranosidases, acetyl xylan esterases, α-glucuronidases, endoxylanases, polygalacturonases, α-galactosidases, acetyl esterases, and combinations thereof and/or the plant biomass is selected from switchgrass, miscanthus, reed canary grass and combinations thereof.

16. The method of claim 15, wherein the enzyme-producing microorganism is *Trichoderma reesei* and the ammonia-pretreated biomass is ammonia fiber expansion (AFEX) pretreated corn stover, wherein a greater than two fold increase in protein abundance in expression of cellulases and hemicellulases is demonstrated as compared to lactose-induced *Trichoderma reesei,* the cellulases are selected from Cel61A and Cel12A, and the hemicellulases are selected from β-xylosidase, endoxylanase, α-arabinofuranosidase, CIP2, acetyl xylan esterase, α-glucuronidase, and polygalaturonase.

## Patentansprüche

1. Verfahren, das umfasst:
Behandeln von Pflanzenbiomasse mittels einer Ammoniumvorbehandlung, um eine ammonium-vorbehandelte Pflanzenbiomasse bereitzustellen, wobei die Pflanzenbiomasse von einer oder mehreren Pflanzen stammt, und wobei die Biomasse lösliche Stoffe oder lösliche Stoffe und Feststoffe enthält;
Extrahieren von zumindest einem Teil der löslichen Stoffe aus der ammoniumvorbehandelten Pflanzenbiomasse mit Hilfe eines Lösungsmittels, um einen löslichen Extrakt und eine extrahierte ammoniumbehandelte Pflanzenbiomasse bereitzustellen, wobei das Lösungsmittel Wasser ist; und
Verwenden des löslichen Extrakts als Stimulans zur Stimulation des Wachstums und zur Förderung der Synthese von Cellulase und/oder Hemicellulase in einem enzymproduzierenden Mikroorganismus.

2. Verfahren gemäß Anspruch 1, wobei der lösliche Extrakt Proteine, Vitamine, einfache Zucker, Oligosaccharide, Lipide, Spurenelemente oder Kombinationen davon umfasst, und/oder wobei der enzymproduzierende Mikroorganismus aus Hefe, Bakterien, Pilzen und Kombinationen davon ausgewählt wird.

3. Verfahren gemäß Anspruch 2, wobei die Ammoniumvorbehandlung Ammoniumfaserexpansion umfasst.

4. Verfahren gemäß Anspruch 1, wobei zumindest eine der einen oder mehreren Pflanzen eine Monokotyledone ausgewählt aus Gras, Maisstroh, Hirse, Zuckerrohr-Bagasse, Weizen, Reis, Mais und einer Kombination davon ist.

5. Verfahren gemäß Anspruch 4, wobei zumindest ein Teil der extrahierten ammoniumvorbehandelten Pflanzenbiomasse als Stimulans an eine Bioprodukt-Produktionseinheit bereitgestellt wird, wobei das Stimulans ein mikrobielles Wachstumsstimulans und/oder ein Agens zur Induktion der Enzymproduktion in einem Mikroorganismus ist, und wobei das Verfahren ferner das Hydrolysieren eines Teils der extrahierten ammoniumvorbehandelten Pflanzenbiomasse mit einem Enzym umfasst, um eine enzymatische Hydrolysat-Aufschlämmung zu erzeugen, wobei das Enzym ein von einem enzymproduzierenden Mikroorganismus segregiertes Enzym ist.

6. Verfahren gemäß Anspruch 5, das ferner aufweist:
mechanisches Verarbeiten der enzymatischen Hydrolysat-Aufschlämmung, um ein flüssiges enzymatisches Hydrolysat zu erzeugen, das ein fermentierbares Zuckergemisch umfasst; und
optionales Fermentieren des flüssigen enzymatischen Hydrolysats, um ein Bioprodukt zu erzeugen.

7. Verfahren gemäß Anspruch 1, wobei die löslichen Stoffe unter alkalischen Bedingungen bei einem pH zwischen 7 und 12 extrahiert werden.

8. Verfahren gemäß Anspruch 1, wobei das Verfahren in einer Bioprodukt-Produktionsanlage durchgeführt wird, wobei die Bioprodukt-Produktionsanlage eine Bioprodukt-Produktionseinheit und eine Enzym-Produktionseinheit aufweist, welche in Verbindung mit der Bioprodukt-Produktionseinheit steht, wobei die Bioprodukt-Produktionsanlage die extrahierte ammoniumvorbehandelte Biomasse verwendet, um ein Bioprodukt in der Bioprodukt-Produktionseinheit zu erzeugen, und den enzymproduzierenden Mikroorganismus in der Enzym-Produktionseinheit enthält, und die Enzym-Produktionseinheit optional die extrahierte ammoniumvorbehandelte Biomasse, ein nähstoffhaltiges Nebenprodukt und/oder den löslichen Extrakt der Bioprodukt-Produktionseinheit enthält.

9. Verfahren gemäß Anspruch 8, wobei das Bioprodukt ein Biokraftstoff oder eine Biochemikalie ist.

10. Verfahren gemäß Anspruch 9, wobei der Biokraftstoff Ethanol ist und wobei die ammoniumvorbehandelte Biomasse ammoniumvorbehandeltes Maisstroh ist.

11. Verfahren gemäß einem der Ansprüche 8 bis 10, wobei das Enzym in einer Enzym-Produktionseinheit erzeugt wird, welche einen enzymproduzierenden Mikroorganismus, eine Rückstand der Ethanolerzeugung und einen löslichen Extrakt enthält.

12. Verfahren gemäß Anspruch 11, wobei die ammoniumvorbehandelte Pflanzenbiomasse ammoniumvorbehandeltes Maisstroh ist, und der Rückstand der Ethanolerzeugung Maisstroh ist.

13. Verfahren gemäß einem der Ansprüche 5 bis 9, das ferner das Kombinieren der ammoniumvorbehandelten Pflanzenbiomasse mit einem Teil der extrahierten ammoniumvorbehandelten Pflanzenbiomasse umfasst, um einen kombinierten Strom vor der Bereitstellung an die Bioprodukt-Produktionseinheit zu erzeugen.

14. Verfahren gemäß Anspruch 13, wobei die Enzym-produktionseinheit ferner ein verdünntes vorbehandeltes Biomasse-Hydrolysat umfasst.

15. Verfahren gemäß Anspruch 14, wobei der enzymproduzierende Mikroorganismus ausgewählt ist aus *Trichoderma reesei, Aspergillus awamori, Clostridium thermocellum* und *Thermoanaerobacterium saccharolyticum,* das Enzym ausgewählt ist aus Exoglucanasen, Endoglucanasen, β-Xylosidasen, Endoxylanasen, α-Arabinofuranosidasen, cellulose-induziertem Protein 2, α-Arabinofuranosidases, Acetyl-xylanesterasen, α-Glucuronidasen, Endoxylanasen, Polygalacturonasen, α-Galactosidasen, Acetylesterasen und Kombinationen, davon und/oder die Pflanzenbiomasse ausgewählt ist aus Rutenhirse, Elefantengras, Rohrglanzgras und Kombinationen davon.

16. Verfahren gemäß Anspruch 15, wobei der enzymproduzierende Mikroorganismus *Trichoderma reesei* ist und die ammoniumvorbehandelte Biomasse mittels Ammoniumfaserexpansion vorbehandeltes Maisstroh ist, wobei im Vergleich zu laktose-induziertem *Trichoderma reesei* eine mehr als zweifache Erhöhung der Proteinexpressionsniveaus von Cellulasen und Hemicellulasen nachweisbar ist, die Cellulasen ausgewählt sind aus Cel61A und Cel12A, und die Hemicellulasen ausgewählt sind aus β-Xylosidase, Endoxylanase, α-Arabinofuranosidase, CIP2, Acetyl-xylanesterase, α-Glucuronidase und Polygalacturonase.

## Revendications

1. Un procédé comprenant:
le traitement de la biomasse de plantes avec un pré-traitement de l'ammoniac pour donner une biomasse de plantes prétraitée ammoniac, dans lequel la biomasse de plantes est d'une ou plusieurs plantes, et dans lequel la biomasse contient des produits solubles ou solubles et des solides;
extraire au moins une partie des substances solubles à partir de la biomasse de plantes prétraitée ammoniac avec une solvant pour produire un extrait soluble et un extrait d'une biomasse de plantes traitée à l'ammoniac, dans lequel le solvant est l'eau; et
l'extrait soluble dans l'utilisation comme stimulant pour stimuler la croissance et la promotion de la synthèse de l'enzyme cellulase et/ou hémicellulase dans un micro-organisme produisant l'enzyme.

2. Procédé selon la revendication 1, dans lequel l'extrait soluble comprend des protéines, des vitamines, des sucres simples, des oligosaccharides, des lipides, des oligo-éléments ou des combinaisons de ceux-ci, et/ou dans lequel le micro-organisme produisant l'enzyme est choisi parmi la levure, des bactéries, des champignons, et des combinaisons de celui-ci.

3. Procédé selon la revendication 2, dans lequel le prétraitement comprend l'expansion de la fibre d'ammonium.

4. Procédé selon la revendication 1, dans lequel au moins une des une ou plusieurs plantes est une monocotylédone choisie parmi l'herbe, les tiges de maïs, le sorgho, la canne à sucre bagasse, le blé, le riz, le maïs, et une combinaison de ceux-ci.

5. Procédé selon la revendication 4, dans lequel au moins une partie de la biomasse de plantes en ammoniac prétraité extrait est fourni à une unité de production de bioproduits comme un stimulant, dans laquelle le stimulant est un stimulant de la croissance microbienne et/ou agent induisant la production d'enzyme à partir d'un micro-organisme, et le procédé comprend en outre l'hydrolyse de la partie de la biomasse de plantes en ammoniac extrait prétraité avec une enzyme pour produire un hydrolysat enzymatique suspension, dans lequel l'enzyme est une enzyme sécrétée par le micro-organisme produisant l'enzyme.

6. Procédé selon la revendication 5, comprenant en outre:
le traitement mécanique du lisier hydrolysat enzymatique pour produire un hydrolysat enzymatique liquide comprenant un mélange de sucre fermentescible; et
fermentation éventuellement l'hydrolysat enzymatique liquide pour générer une bioproduits.

7. Procédé selon la revendication 1, dans lequel les matières solubles sont extraites dans des conditions alcalines ayant un pH compris entre 7 et 12.

8. Procédé selon la revendication 1, dans lequel le procédé est exécuté dans une installation de production de bioproduits, la facilité de production de bioproduits, y compris une unité de production de bioproduits et une unité de production d'enzyme en communication avec l'unité de production de bioproduits, dans lequel l'installation de production de bioproduit utilise la biomasse de l'ammoniac prétraité extrait pour produire un bioproduit dans l'unité de production de bioproduits et contenant le micro-organisme produisant l'enzyme dans l'unité de production de l'enzyme, l'unité de production d'enzymes comprenant éventuellement l'extrait ammoniac prétraité la biomasse, un sous-produit contenant des éléments nutritifs et/ou de l'extrait soluble dans l'unité de production de bioproduits.

9. Procédé selon la revendication 8, dans lequel le produit biologique est un biocarburant ou biochimique.

10. Procédé selon la revendication 9, dans laquelle le biocarburant est de l'éthanol et de l'ammoniac est l'ammoniac biomasse prétraitée prétraité les tiges de maïs.

11. Procédé selon l'une quelconque des revendications 8 à 10, dans lequel l'enzyme est produite dans une unité de production d'enzyme comprenant un micro-organisme produisant l'enzyme, un résidu de la production d'éthanol, et l'extrait soluble.

12. Procédé selon la revendication 11, dans lequel la biomasse de plantes prétraitée ammoniac est l'ammoniac tiges de maïs prétraitées et le résidu de la production d'éthanol est la canne de maïs.

13. Procédé selon l'une quelconque des revendications 5 à 9, comprenant en outre la combinaison de la biomasse de plantes ammoniac prétraité avec la partie de la biomasse végétale en ammoniac prétraité extrait pour former un courant combiné avant de fournir à l'unité de production de bioproduits.

14. Procédé selon la revendication 13, dans lequel l'unité de production d'enzyme comprend en outre un hydrolysat de la biomasse prétraitée dilué.

15. Le procédé de la revendication 14, dans lequel le micro-organisme produisant l'enzyme est sélectionnée à partir de *Trichoderma reesei, Aspergillus awamori, Clostridium thermocellum* et *Thermoanaerobacterium saccharolyticum,* l'enzyme est choisie dans exoglucanases, les endoglucanases, les β-xylosidases, endoxylanases, des α-arabinofuranosidases, de la cellulose protéine induite 2, α-arabinofuranosidases, estérases d'acétyle xylane, les α-glucuronidases, endoxylanases, polygalacturonases, α-galactosidases, estérases d'acétyle, et leurs combinaisons et/ou la biomasse de plantes est sélectionné à partir du panic érigé, le miscanthus, l'alpiste roseau et leurs combinaisons.

16. Procédé selon la revendication 15, dans lequel le micro-organisme produisant l'enzyme est *Trichoderma reesei* et la biomasse est l'expansion de la fibre d'ammonium (AFEX) prétraité tiges de maïs prétraitée, dans lequel une augmentation d'un facteur supérieur à deux en protéine abondance de l'expression de cellulases et hémicellulases est démontrée par comparaison avec induite lactose *Trichoderma reesei,* les cellulases sont choisies parmi Cel61A et Cel12A, les hémicellulases et sont choisis parmi les β-xylosidase, endoxylanase, α-arabinofuranosidase, CIP2, estérase d'acétyle xylane, α-glucuronidase, et polygalacturonase.
